(19) Europäisches Patentamt
European Patent Office
Office européen des brevets

(11) **EP 3 955 008 A1**

(12) **EUROPEAN PATENT APPLICATION**
published in accordance with Art. 153(4) EPC

(43) Date of publication:
**16.02.2022 Bulletin 2022/07**

(21) Application number: **20788579.9**

(22) Date of filing: **28.01.2020**

(51) International Patent Classification (IPC):
**G01N 35/10** (2006.01)   **G06T 7/00** (2017.01)

(52) Cooperative Patent Classification (CPC):
**G01N 35/10; G06T 7/00**

(86) International application number:
**PCT/JP2020/002860**

(87) International publication number:
**WO 2020/208899 (15.10.2020 Gazette 2020/42)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **08.04.2019   JP 2019073334**

(71) Applicant: **Hitachi High-Tech Corporation**
**Minato-ku**
**Tokyo 105-6409 (JP)**

(72) Inventors:
• **KAKISHITA, Yasuki**
 **Tokyo 100-8280 (JP)**
• **HATTORI, Hideharu**
 **Tokyo 100-8280 (JP)**
• **SAKAZUME, Taku**
 **Tokyo 105-6409 (JP)**
• **SUZUKI, Yoichiro**
 **Tokyo 105-6409 (JP)**

(74) Representative: **MERH-IP Matias Erny Reichl Hoffmann**
**Patentanwälte PartG mbB**
**Paul-Heyse-Strasse 29**
**80336 München (DE)**

(54) **IMAGE PROCESSING DEVICE, AUTOMATIC ANALYSIS SYSTEM, AND IMAGE PROCESSING METHOD**

(57)     Provided is an image processing apparatus, an automatic analysis system, and an image processing method capable of determining a state of a container or a state of a collection target based on an image acquired by a single camera. The image processing apparatus includes: an image acquisition unit configured to acquire an upper image that is an image obtained by capturing an image of a container accommodating a collection target including a sample, a reagent, and a reaction solution from above; a region calculation unit configured to calculate an edge region of the container or an upper surface region of the collection target from the upper image; and a state determination unit configured to determine a state of the container or a state of the collection target based on the edge region or the upper surface region.

FIG. 2

EP 3 955 008 A1

**Description**

BACKGROUND OF THE INVENTION

1. Field of the Invention

The present invention relates to a technique of determining a state of a container in which a collection target such as a sample, a reagent, or a reaction solution is stored or a state of the collection target in an automatic analysis system including an immune analyzing apparatus or the like.

2. Description of the Related Art

In an automatic analysis system including an immune analyzing apparatus or the like, in order to analyze components of a sample such as blood and urine, a state of color development or light emission generated from a reaction solution obtained by reacting the sample with a reagent is measured. The sample, the reagent, the reaction solution, and the like used for analysis are stored in a container such as a test tube, and are collected from the container by a collection unit such as a dispensing probe. The container containing the sample, the reagent, the reaction solution, and the like, which are collection targets of the collection unit, has a plurality of types of different inner diameters and lengths, and it is desirable that the type of the container is automatically determined in a situation in which a plurality of containers are mixed.

JP-A-2004-151025 (PTL 1) discloses that a type of a container is determined by comparing a figure obtained by imaging the container from two directions of an opening portion direction and a side surface direction with a standard figure stored in a memory.

However, in PTL 1, in order to determine the type of the container, a plurality of cameras that capture images of the container from the two directions, that is, the opening portion direction and the side surface direction, are required, which increases a hardware cost. In PTL 1, only the determination of the type of the container is performed, and the determination of the state of the container or the collection target, such as an inclination of the container or an amount of the collection target is not considered.

An object of the invention is to provide an image processing apparatus, an automatic analysis system, and an image processing method capable of determining a state of a container or a collection target based on an image acquired by a single camera.

SUMMARY OF THE INVENTION

In order to achieve the above object, the invention provides an image processing apparatus including: an image acquisition unit configured to acquire an upper image that is an image obtained by capturing an image of a container accommodating a collection target including a sample, a reagent, and a reaction solution from above; a region calculation unit configured to calculate an edge region of the container or an upper surface region of the collection target from the upper image; and a state determination unit configured to determine a state of the container or a state of the collection target based on the edge region or the upper surface region.

The invention further provides an automatic analysis system including the image processing apparatus, automatic analysis system including: a collection unit configured to collect the collection target from the container; and a control unit configured to control the collection unit based on a determination result of the state determination unit.

The invention further provides an image processing method including: an image acquiring step of acquiring an upper image that is an image obtained by capturing an image of a container accommodating a collection target including a sample, a reagent, and a reaction solution from above; a region calculating step of calculating an edge region of the container or an upper surface region of the collection target from the upper image; and a state determination step of determining a state of the container or a state of the collection target based on the edge region or the upper surface region.

The invention further provides an image processing method for dividing an image into a plurality of regions, the method including: creating a dilate kernel by inserting a predetermined number of zeros between elements of a kernel used in a convolution processing and a pooling processing; and executing the convolution processing and the pooling processing by using the dilate kernel.

According to the invention, an image processing apparatus, an automatic analysis system, and an image processing method capable of determining a state of a container or a collection target based on an image acquired by a single camera can be provided.

BRIEF DESCRIPTION OF THE DRAWINGS

FIG. 1 is a diagram showing an example of a hardware configuration of an automatic analysis system;
FIG. 2 is an example of a functional block diagram according to a first embodiment;
FIG. 3 is a diagram showing an example of a processing flow according to the first embodiment;
FIG. 4 is a diagram showing an example of a result of a region segmentation processing;
FIG. 5 is a diagram showing calculation of an edge region of a container;
FIG. 6A is a diagram showing an example of a table used for determining a type of the container;
FIG. 6B is a diagram showing an example of a table for obtaining a length of the container and the like from the type of the container;
FIG. 7 is a diagram showing a schematic convolution processing;
FIG. 8 is a diagram showing a schematic pooling processing;
FIG. 9 is a diagram showing an example of a region segmentation processing by fully convolutional networks (FCN) ;
FIG. 10 is a diagram showing an example of a high-resolution region segmentation processing by FCN;
FIG. 11 is a diagram showing an example of a kernel in a convolution processing and a dilated convolution processing.
FIG. 12 is a diagram showing an example of a kernel in a pooling processing and a dilated pooling processing;
FIG. 13 is a diagram showing a schematic dilate processing;
FIG. 14 is a diagram showing an example of a procedure of region segmentation using a dilate kernel;
FIG. 15 is a diagram showing the region segmentation using the dilate kernel;
FIG. 16 is a diagram showing an example of a processing flow according to a second embodiment;
FIG. 17 is a diagram showing calculation of an upper surface region of a collection target;
FIG. 18A is a diagram showing an example of a non-telecentric upper image;
FIG. 18B is a diagram showing an example of a telecentric upper image;
FIG. 18C is a diagram showing an example of a telecentric oblique image;
FIG. 19 is a diagram showing an example of a table for obtaining a distance from an edge of a container to an upper surface of the collection target;
FIG. 20 is a diagram showing an example of a processing flow according to a third embodiment;
FIG. 21A is a diagram showing inclination determination based on the non-telecentric upper image;
FIG. 21B is a diagram showing inclination determination based on the telecentric upper image;
FIG. 22A is a diagram showing calculation of an inclination angle based on the non-telecentric upper image;
FIG. 22B is a diagram showing calculation of an inclination angle based on the telecentric upper image;
FIG. 23 is an example of a functional block diagram according to a fourth embodiment;
FIG. 24 is a diagram showing an example of a processing flow according to the fourth embodiment;
FIG. 25 is a diagram showing an example of a screen displaying a list of images to which teacher signals are assigned;
FIG. 26 is a diagram showing an example of an operation screen for specifying a region to which a teacher signal is to be assigned; and
FIG. 27 is a diagram showing an example of an operation screen for assigning the teacher signal.

DESCRIPTION OF THE PREFERRED EMBODIMENTS

Preferred embodiments of an image processing apparatus, an automatic analysis system, and an image processing method according to the invention will be described below with reference to accompanying drawings. In the following description and the accompanying drawings, components having the same function and structure are denoted by the same reference numerals, and repeated description thereof will be omitted.

[First Embodiment]

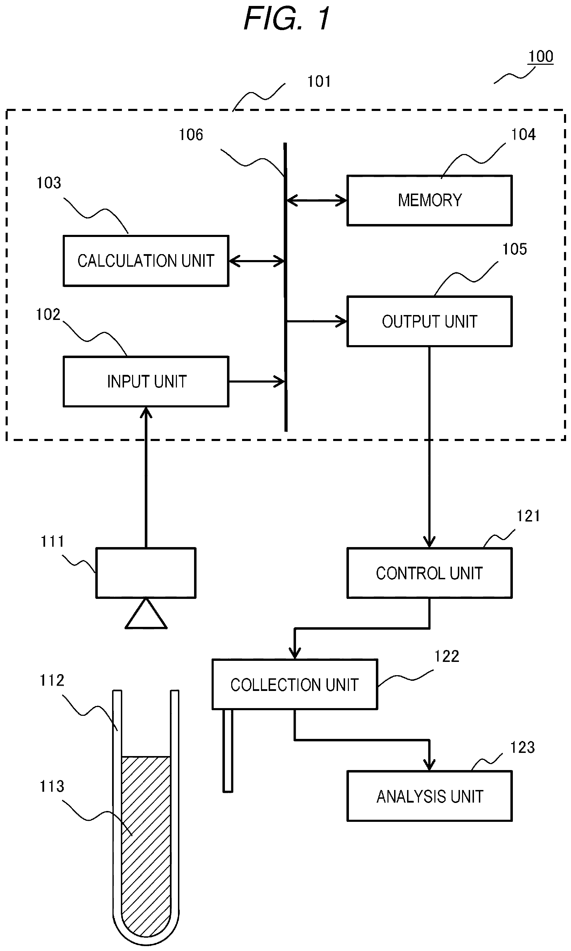

An example of a hardware configuration of an automatic analysis system 100 according to the present embodiment will be described with reference to FIG. 1. The automatic analysis system 100 includes an image processing apparatus 101, an imaging unit 111, a control unit 121, a collection unit 122, and an analysis unit 123. Specifically, the image processing apparatus 101 includes an input unit 102, a calculation unit 103, a memory 104, and an output unit 105, and is connected to the imaging unit 111 and the control unit 121. The image processing apparatus 101 may be provided in a system other than the automatic analysis system 100. Hereinafter, each unit will be described.
The imaging unit 111 is a device that images a container 112 and a collection target 113 stored in the container 112, and is, for example, a camera. The container 112 is a test tube or the like of a cylindrical shape or a tapered shape, and has a bottom surface at one end and an opening portion at the other end. The container 112 has a plurality of types of different inner diameters and lengths. The collection target 113 is a sample such as blood or urine, a reagent to be reacted with the sample, a reaction solution obtained by reacting the sample with the reagent, or the like, and is a liquid or powder. The imaging unit 111 is a single unit, is installed on an opening portion side of the container 112, that is, above the container 112, and images the container 112 and the collection target 113 from above the container 112. An image captured from above the container 112 by the imaging unit 111 is referred to as an upper image. A telecentric or non-telecentric lens is attached to the imaging unit 111.

The input unit 102 is an interface that receives data of an upper image from the imaging unit 111 or a recording device in which the upper image is recorded, and receives an operation signal generated by an operator operating a keyboard, a mouse, or the like. The upper image may be a still image such as BMP, PNG, or JPEG, or may be a frame image extracted from a moving image such as MPEG or H.264 at regular intervals.

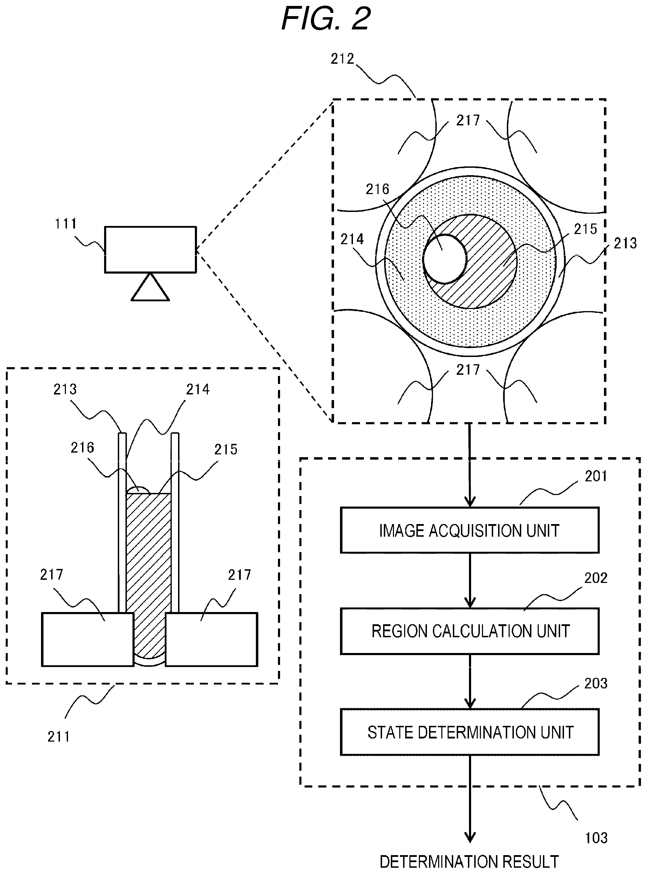

The calculation unit 103 is a device that executes various processing in the image processing apparatus 101, and is, for example, a central processing unit (CPU) or a field-programmable gate array (FPGA). Functions executed by the calculation unit 103 will be described later with reference to FIG. 2.

The memory 104 is a device that stores programs executed by the calculation unit 103, parameters, coefficients, processing results, and the like, and is a HDD, a RAM, a ROM, a flash memory, or the like.

The output unit 105 is an interface that transmits a result of the processing by the calculation unit 103 to the outside, and outputs the result of the processing to, for example, a display device such as a display or a printer, a recording device that records the result of the processing, or a control device that controls other devices. FIG. 1 shows an example in which the result of processing is output to the control unit 121, which is an example of a control device that controls other devices.

The control unit 121 is a device that controls the operation of the collection unit 122 based on the result of the processing output from the output unit 105, and is specifically a central processing unit (CPU) or the like.

The collection unit 122 is a device that collects the collection target 113 from the container 112 by the control unit 121, and is, for example, a dispensing probe.

The analysis unit 123 is a device that performs analysis using the collection target 113, and is, for example, an immune analyzing apparatus. A result of the analysis by the analysis unit 123 is displayed on a display or the like.

An example of a functional block diagram of the present embodiment will be described with reference to FIG. 2. The functions may be configured with dedicated hardware, or may be configured with software operating on the calculation unit 103. The present embodiment includes an image acquisition unit 201, a region calculation unit 202, and a state determination unit 203. Hereinafter, each unit will be described.

The image acquisition unit 201 acquires an upper image 212 captured by the imaging unit 111. The upper image 212 includes an edge 213 and an inner wall 214 of the container 112, an upper surface 215 of the collection target 113, a mixture 216, and a fixing unit 217. The mixture 216 is bubbles, lipids, or the like present inside or on the upper surface of the collection target 113. The fixing unit 217 is a holder or the like that fixes the container 112. For each part included in the upper image 212, reference is also made to a side view 211.

The region calculation unit 202 calculates a region of each portion, for example, a region of the edge 213 of the container 112 or a region of the upper surface 215 of the collection target 113, from the upper image 212 acquired by the image acquisition unit 201. In order to calculate the region, threshold processing using a luminance value of an image, region segmentation processing using fully convolutional networks (FCN), and the like are executed.

The state determination unit 203 determines states of the container 112 and the collection target 113, for example, a type of the container 112, an inclination of the container 112, and an amount of the collection target 113, based on one of the regions calculated by the region calculation unit 202.

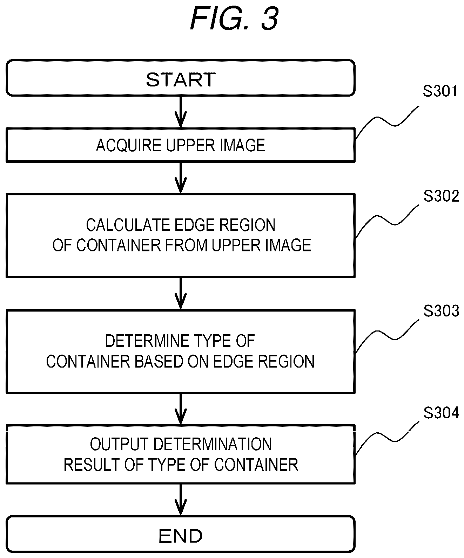

An example of a processing flow of the present embodiment will be described with reference to FIG. 3. In the present embodiment, the type of the container 112 is determined based on the region of the edge 213 of the container 112 calculated from the upper image 212.

(S301)

The image acquisition unit 201 acquires the upper image 212 via the input unit 102. The acquired upper image 212 may be an image captured by the imaging unit 111 or an image recorded in a recording device or the like (not shown).

(S302)

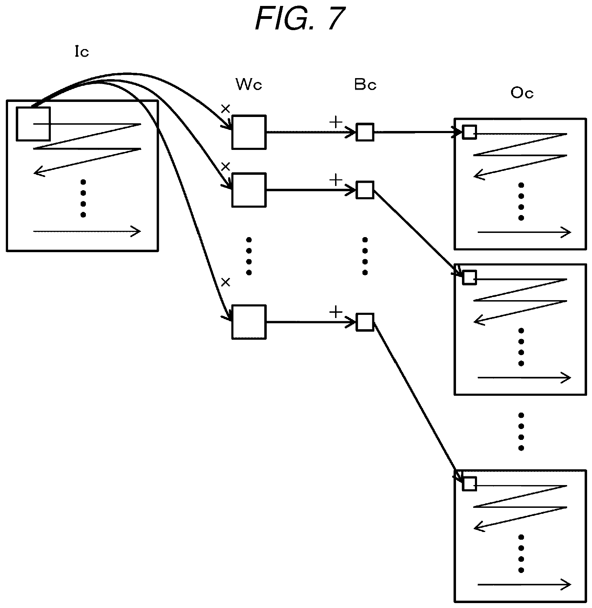

The region calculation unit 202 calculates a region of the edge 213 of the container 112 from the upper image 212 acquired in S301. In order to calculate the region of the edge 213, one of the threshold processing using the luminance value of the upper image 212, region segmentation processing using FCN, or the like is executed. The region segmenting processing using FCN will be described later with reference to FIGS. 7 to 15.

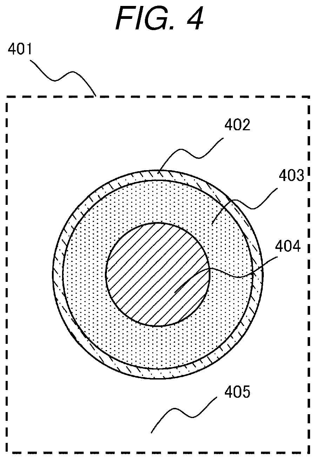

An example of a region segmentation image 401 obtained by performing the region segmentation processing on the upper image 212 will be described with reference to FIG. 4. The region segmentation image 401 includes an edge region 402, an inner wall region 403, an upper surface region 404, and a background region 405. The edge region 402 is a region of the edge 213 of the container 112. The inner wall region 403 is a region of the inner wall 214 of the container 112 and is a region between the edge 213 of the container 112 and the collection target 113. The upper surface region 404 is a region of the upper surface 215 of the collection target 113, and the mixture 216 is also included in the upper surface region 404. The background region 405 is the entire region outside the edge region 402, and the fixing unit 217 is also included in the background region 405.

(S303)

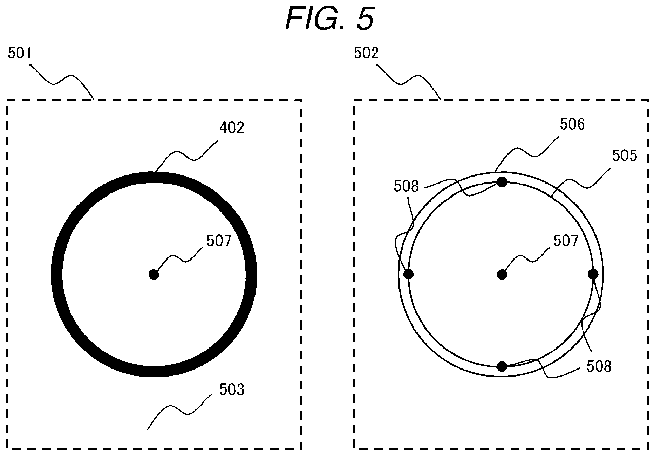

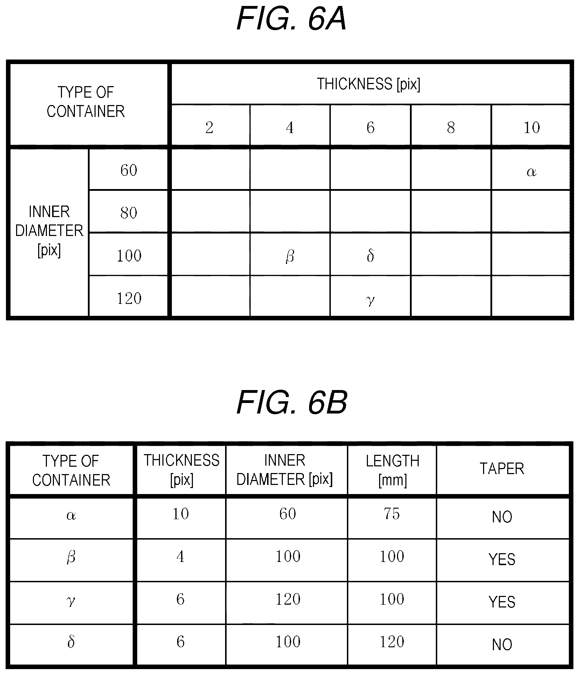

The state determination unit 203 determines the type of the container 112 based on the edge region 402 calculated in S302. The step will be described with reference to FIGS. 5 and 6.

The state determination unit 203 creates a binarized image 501 of the edge based on the calculation result in S302. The edge binarized image 501 includes the edge region 402 and a region 503 other than the edge region 402. For example, a luminance value of 0 is given to the edge region 402, and a luminance value of 255 is given to the other region 503.

The state determination unit 203 extracts a boundary of the edge region 402 by applying a Sobel filter or the like to the binarized image 501 of the edge, and creates a boundary image 502 of the edge. Since the edge 213 of the container 112 has an annular shape, the boundary image 502 of the edge includes an inner boundary 505, which is a boundary on an inner wall side of the container 112, and an outer boundary 506, which is a boundary on an outer wall side of the container 112.

The state determination unit 203 performs elliptical fitting on the inner boundary 505 and the outer boundary 506, calculates long diameters of the inner boundary 505 and the outer boundary 506, sets the long diameter of the inner boundary 505 as an inner diameter of the container 112, and obtains a thickness of the container 112 from the long diameters of the inner boundary 505 and the outer boundary 506 using the following equation.

[Equation 1]

$$D = (Lo - Lc)/2$$

Here, D is the thickness of the container 112, Lo is the long diameter of the outer boundary 506, and Lc is the long diameter of the inner boundary 505.

A reason why the long diameter is used instead of a short diameter is to reduce the influence of an inclination of the container 112. That is, as the container 112 is inclined from an upright state, short diameters of the inner boundary 505 and the outer boundary 506 become shorter, while the long diameters are less likely to change. For the elliptical fitting, all points of the inner boundary 505 or the outer boundary 506 may be used, or at least three extraction points 508 arbitrarily extracted from the inner boundary 505 or the outer boundary 506 may be used. As the extraction point 508, for example, a point reaching the inner boundary 505 or the outer boundary 506 is extracted by scanning in upward, downward, left, and right directions from a center of gravity 507 obtained by arithmetically averaging coordinates of the edge region 402.

The state determination unit 203 determines the type of the container 112 by comparing the inner diameter and the thickness of the container 112 obtained from the long diameters of the inner boundary 505 and the outer boundary 506 with a table shown in FIG. 6A. For example, when the obtained inner diameter of the container 112 is 60 pixels and the thickness thereof is 10 pixels, it is determined that the type of the container is α. An item in the table corresponding to an obtained inner diameter and thickness may be determined by an absolute value of a difference between the obtained value and the numerical value of each item. For example, when the obtained inner diameter is 98 pixels and the thickness is 6 pixels, the inner diameter of 100 pixels and the thickness of 6 pixels are selected from the table, and it is determined that the type of the container 112 is δ.

The table shown in FIG. 6A is created in advance by measuring inner diameters and thicknesses of a plurality of types of containers 112, and is stored in the memory 104 or the like. A blank space in the table indicates an unknown container, and when the inner diameter and the thickness obtained in the step correspond to the blank space, it is determined that the container 112 included in the upper image 212 is an unknown container.

(S304)

The output unit 105 outputs the type of the container 112 determined in S303. The result of the determination is transmitted to, for example, the control unit 121, and the control unit 121 controls the collection unit 122 based on the result of the determination. For example, when it is determined that the container 112 is an unknown container, the control unit 121 may stop the collection unit 122. When the container 112 is an unknown container, collision between the collection unit 122 and the container 112 can be avoided by stopping the collection unit 122.

The control unit 121 may adjust the height of the collection unit 122 according to the type of the container 112. For example, the height of the collection unit 122 may be adjusted based on the length of the container 112 acquired by comparing the type of the container 112, which is the determination result, with a table shown in FIG. 6B. The table shown in FIG. 6B is created in advance by measuring inner diameters and thicknesses, lengths, and presence or absence of taper of a plurality of types of containers 112, and is stored in the memory 104 or the like, and reference is made as necessary.

The output unit 105 may output the determination result to a display or a printer to present the type of the container 112 to the operator, or may display a message prompting the operator to change the container 112 when the container 112 is an unknown container. Further, the output unit 105 may output the type of the container 112 to another image processing apparatus, and may output an image on which a correction processing according to the type of the container 112 is performed together with the type of the container 112. The correction processing according to the type of the container 112 is, for example, processing of normalizing the image size according to the inner diameter of the container 112 or processing of correcting the contrast according to the type of the container 112.

According to the processing flow described above, the image processing apparatus 101 can determine the type of the container 112, which is one of the states of the container 112, based on the upper image 212 acquired by the single imaging unit 111. The automatic analysis system 100 can appropriately control the collection unit 122 according to the determination result by the image processing apparatus 101, and thus can perform more accurate analysis without reducing an inspection efficiency.

Here, the region segmentation processing using FCN will be described. The FCN is one of deep neural network (DNN), and is a region segmentation processing of segmenting an image into regions by constructing all layers with convolutional neural network (CNN). The CNN includes three pieces of processing, that is, a convolution processing, a pooling processing, and an activation processing. The convolution processing is processing of creating a map of a feature amount from input data, the pooling processing is processing of extracting a representative value from the input data, and the activation processing is processing of applying a nonlinear function to the input data.

With reference to FIG. 7, an example of the operation of the convolution processing will be described. As shown in FIG. 7, in the convolution processing, while sliding positions at which luminance values of a plurality of pixels are acquired from an upper left to a lower right of input data Ic, output data Oc is calculated by multiplying the acquired luminance values of the plurality of pixels by a coefficient Wc and adding a coefficient Bc.

Equation 2 shows an example of a calculation equation of the convolution processing.

[Equation 2]

$$Oc(d, y, x) = \sum_{ch} \sum_{fy} \sum_{fx} \bigl( Ic(ch, y * Sy + fy, x * Sx + fx) \times Wc(d, ch, fy, fx) \bigr) + Bc(d)$$
$$where\ fy \in [0, Fy], fx \in [0, Fx]$$

Here, the input data Ic is data having three dimensions of a channel ch, a vertical direction position y, and a horizontal direction position x, and the output data Oc is data having three dimensions of a feature amount number d, a vertical direction position y, and a horizontal direction position x. The multiplication coefficient Wc is a coefficient having four dimensions of the feature amount number d, the channel ch, a vertical direction position fy, and a horizontal direction position fx, and is also referred to as a kernel, and an addition coefficient Bc is a coefficient having a dimension of the feature amount number d. The size of the kernel is represented by a kernel height Fy and a kernel width Fx, and an amount by which the kernel slides per time is represented by a vertical direction stride amount Sy and a horizontal direction stride amount Sx.

The multiplication coefficient Wc and the addition coefficient Bc are obtained in advance by machine learning using a stochastic gradient descent method or the like using a teacher image, which is an image obtained by correctly segmenting each region, as input data, and are stored in the memory 104 as coefficients of the region segmentation processing. That is, machine learning is performed using a teacher signal correctly indicating which of the edge region 402, the inner wall region 403, the upper surface region 404, and the background region 405 each pixel of the plurality of upper images 212 belongs to.

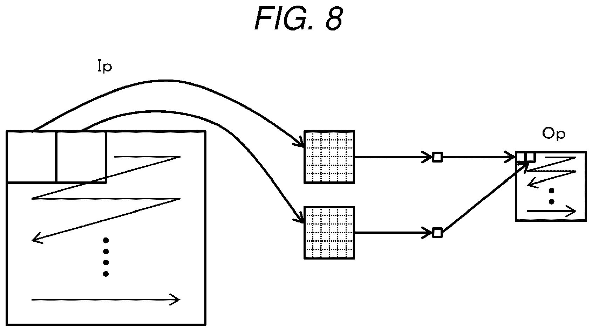

With reference to FIG. 8, an example of the operation of the pooling processing will be described. As shown in FIG. 8, in the pooling processing, output data Op is calculated by extracting a representative value from the acquired luminance values of the plurality of pixels while sliding the positions at which the luminance values of the plurality of pixels are acquired from the upper left to the lower right of input data Ip at a constant pitch. As the representative value, for example, a maximum value or an average value of the luminance values of the plurality of pixels is used, and the representative value extracted in the present embodiment is the maximum value.

Equation 3 shows an example of a calculation equation of the pooling processing.

[Equation 3]

$$Op(ch, y, x) = \max \left( Ip(ch, y * Sy + fy, x * Sx + fx) \right)$$
$$where \ fy \in [0, Fy], fx \in [0, Fx]$$

Here, the input data Ip and the output data Op are data having three dimensions of a channel ch, a vertical direction position y, and a horizontal direction position x. Also in the pooling processing, similar to the convolving processing, the acquired luminance values of the plurality of pixels are multiplied by a coefficient called a kernel, and the size of the kernel is represented by Fy and Fx, and stride amounts of the kernel is represented by Sy and Sx. In the pooling processing, Sy = Fy and Sx = Fx are often set, and all elements of the kernel are 1. The kernel used for the pooling processing is referred to as a P kernel, and the kernel used for the convolution processing is referred to as a C kernel.

The activation processing is processing of applying nonlinear functions of Equation 4 and Equation 5 to the output data Oc of the convolution processing and the output data Op of the pooling processing, and identifying whether a certain pixel is the edge region 402, for example.

[Equation 4]

$$tanh(a) = \frac{e^a - e^{-a}}{e^a + e^{-a}}$$

[Equation 5]

$$ReLU(a) = max(0, a)$$

The CNN obtains a feature amount and an identification result by combining the above-described convolution processing, pooling processing, and activation processing. The FCN is a region segmentator including only CNN, and to which an image of an arbitrary size can be input.

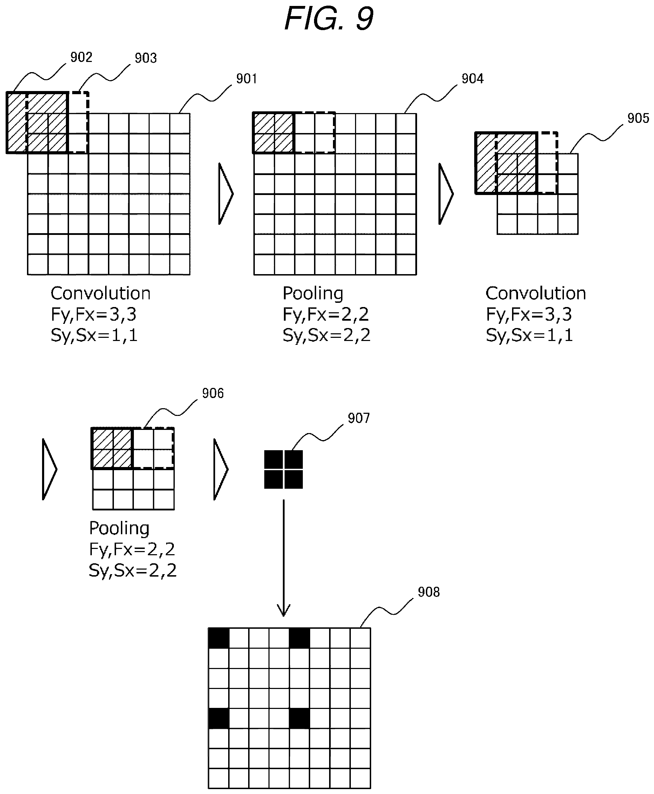

FIG. 9 shows an example of the region segmenting processing by FCN. It is assumed that an input image 901 is an image input to the FCN and has a width and a height of 8 pixels. A first kernel 902 indicates a first processing target region in each layer. A shaded part represents a processing target location. A second kernel 903 indicates a second processing target region in each layer. An amount of deviation between the first kernel 902 and the second kernel 903 is determined by the stride amounts Sy and Sx.

A first layer output data 904 is a result of applying the convolution processing to the input image 901, and a second layer output data 905 is a result of applying the pooling processing to the first layer output data 904. A third layer output data 906 is a result of applying the convolution processing to the second layer output data 905, and a fourth layer output data 907 is a result of applying the pooling processing to the third layer output data 906. Further, a region segmentation result 908 indicates which position in the input image the fourth layer output data 907 corresponds to. Kernel sizes Fy and Fx and the stride amounts Sy and Sx of the convolution processing and the pooling processing are as shown in FIG. 9.

As shown in FIG. 9, each time the processing in which the stride amount is 2 or more is executed, a size of the output data of each layer is reduced according to the stride amount. The fourth layer output data 907, which is a final output, is reduced to 2 × 2 pixels, and only four locations shown in black squares of the region segmentation result 908 are applied to the input image 901. That is, only an identification result of the black squares of the region segmentation result 908 is obtained, and a white square portion is in a state where no identification result is obtained and the region segmentation processing of low resolution is performed.

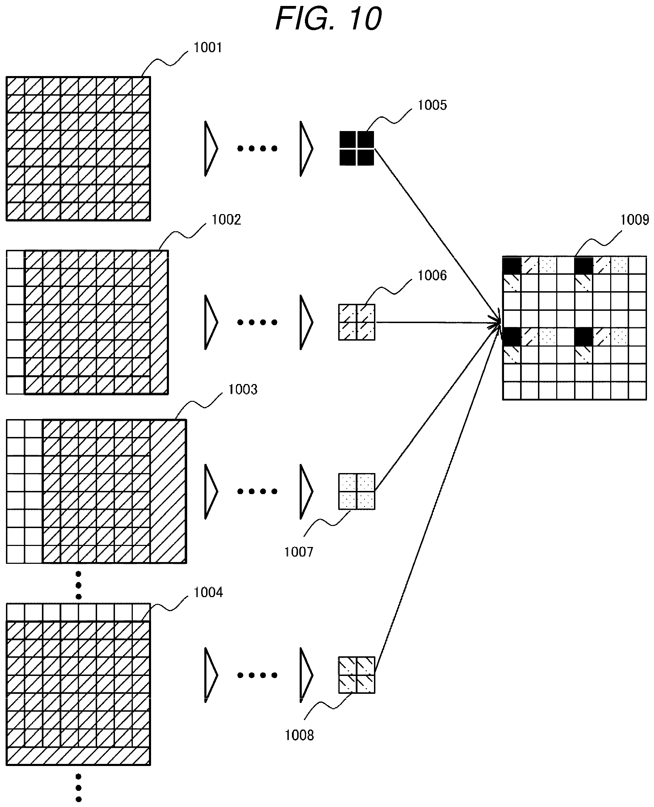

The high-resolution region segmentation processing will be described with reference to FIG. 10. In FIG. 10, images at different acquisition positions are input to the FCN, and a region segmentation result for each image is acquired. That is, a first input image 1001, a second input image 1002, a third input image 1003, a fourth input image 1004, and so on are input, and a first region segmentation result 1005, a second region segmentation result 1006, a third region segmentation result 1007, a fourth region segmentation result 1008, and so on are acquired. The first input image 1001 is the same image as the input image 901 in FIG. 9, and the second input image 1002 is an image obtained by sliding the input image 901 to the right by one pixel, the third input image 1003 is an image obtained by sliding the input image 901 to the right by two pixels, and the fourth input image 1004 is an image obtained by sliding the input image 901 below by one pixel. Due to the sliding of the pixel, the luminance value 0 or the luminance value of an adjacent pixel is embedded in a portion where no pixel exists. By integrating a plurality of acquired region segmentation results, an integrated region segmentation result 1009 is obtained. According to the region segmentation processing of FIG. 10, although a high-resolution region segmentation result can be acquired, the number of times of the region segmentation processing is increased, for example, 16 times in the example of FIG. 10, and thus the processing time is also increased.

Therefore, in the present embodiment, the kernel used for the convolution processing and the pooling processing of the FCN is changed by the dilate processing, and the high-resolution region segmentation result is acquired by one region segmentation processing. Dilate processing is processing of inserting a predetermined number of zeros between elements of a kernel and expanding the kernel, and the kernel after the dilate processing is referred to as a dilate kernel or a D kernel. The convolution processing and the pooling processing using the D kernel are referred to as "dilated convolution processing" and "dilated pooling processing".

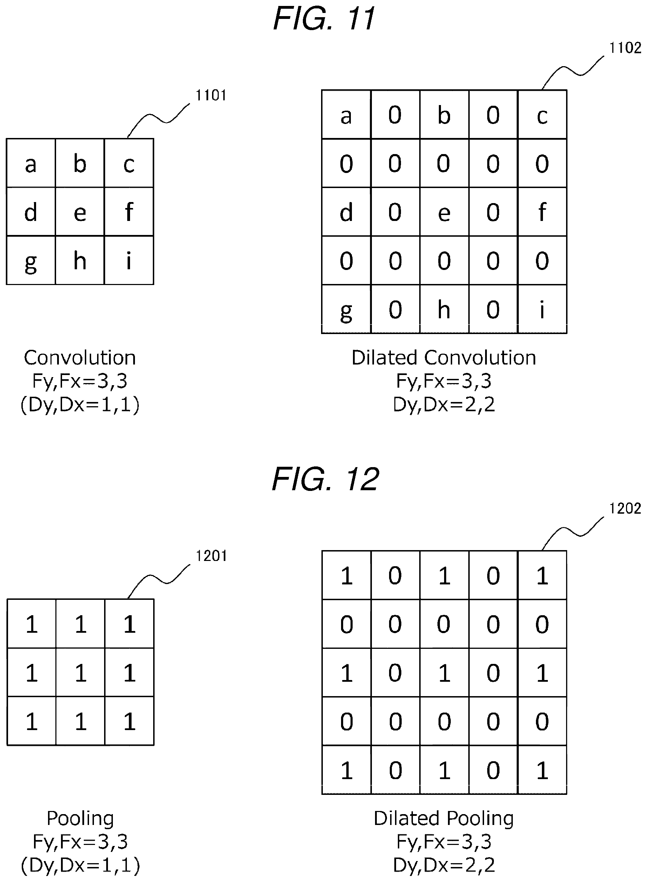

With reference to FIG. 11, an example of the D kernel in the dilated convolution processing will be described. A C kernel 1101 is an example of a kernel used for the convolution processing, and includes nine elements a to i. A DC kernel 1102 is an example of a kernel used for the dilated convolution processing, and is constructed by inserting one zero into adjacent elements of the C kernel 1101. Values obtained by adding 1 to the number of zeros to be inserted are referred to as dilate amounts Dy and Dx, and the DC kernel 1102 in FIG. 11 has Dy and Dx = 2 and 2.

Equation 6 shows an example of a calculation equation of the dilated convolution processing.

[Equation 6]

$$Oc(d, y, x) = \sum_{ch} \sum_{fy} \sum_{fx} \left( Ic(ch, y * Sy + fy * Dy, x * Sx + fx * Dx) \times Wc(d, ch, fy, fx) \right) + Bc(d)$$
$$where\ fy \in [0, Fy], fx \in [0, Fx]$$

In Equation 6, if Dy and Dx = 1 and 1, Equation 2 is obtained.

With reference to FIG. 12, an example of the D kernel in the dilated pooling processing will be described. AP kernel 1201 is an example of a kernel used for the pooling processing, and all nine elements are constructed by 1. A DP kernel 1202 is an example of a kernel used for the dilated pooling processing, and is constructed by inserting one zero into adjacent elements of the P kernel 1201. The dilate amounts Dy and Dx are similar as those in the dilated convolution processing, and the DP kernel 1202 in FIG. 12 is also Dy and Dx = 2 and 2.

Equation 7 shows an example of a calculation equation of the dilated pooling processing.

[Equation 7]

$$Op(ch, y, x) = \max(Ic(ch, y * Sy + fy * Dy, x * Sx + fx * Dx)$$
$$where\ fy \in [0, Fy], fx \in [0, Fx]$$

In Equation 7, if Dy and Dx = 1 and 1, Equation 3 is obtained.

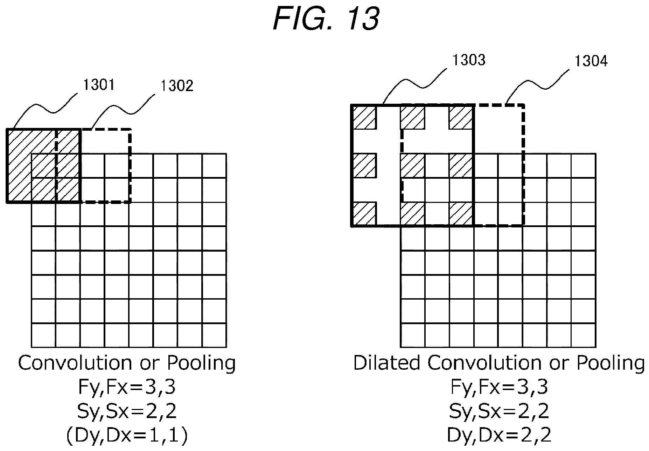

The Dilated convolution processing and the dilated pooling processing will be described with reference to FIG. 13. A first kernel 1301 and a second kernel 1302 are kernels used for the convolution processing or the pooling processing. A first D kernel 1303 and a second D kernel 1304 are kernels used for the dilated convolution processing or the dilated pooling processing. The kernel sizes Fy and Fx are both 3, and the stride amounts Sy and Sx are both 2.

The convolution processing or the pooling processing corresponds to the dilated convolution processing or the dilated pooling processing when both the dilate amounts Dy and Dx are set to 1. The dilate amounts Dy and Dx of the first D kernel 1303 and the second D kernel 1304 are both 2.

A processing range of each kernel may include a region outside the input data. For example, in the case of the second kernel 1302, a first row corresponds to a region outside the input data. In the present embodiment, the luminance value of 0 is embedded in the region outside the input data. The FCN using the dilated convolution processing and the dilated pooling processing is referred to as a dilated FCN (DFCN).

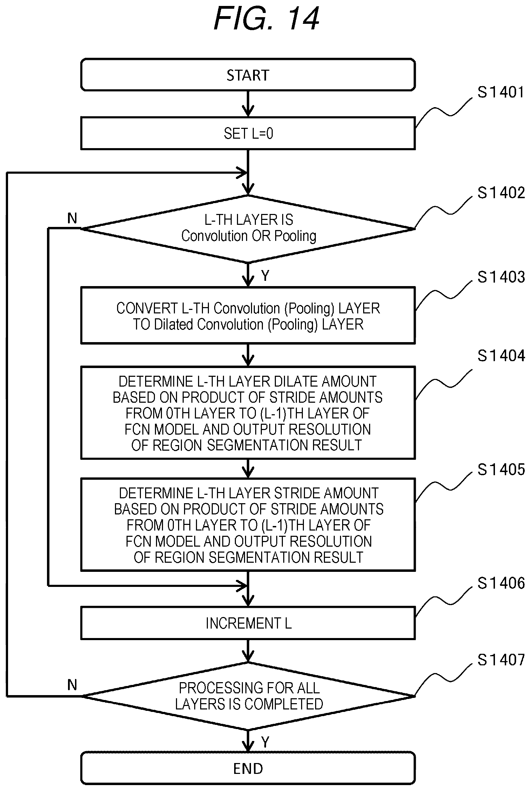

An example of a processing flow of converting a FCN model into a DFCN model will be described with reference to FIG. 14. In the processing flow of FIG. 14, the FCN model is input, and a DFCN model for calculating a region segmentation result in units of Ky pixels in the vertical direction and Kx pixels in the horizontal direction is output. Ky and Kx are higher in resolution as they approach 1, and the larger the values are, the lower the resolution is, and the higher the processing speed is. Here, Ky and Kx are assumed to be one of divisors of products Psy and Psx of stride amounts of all layers in the FCN model. It is assumed that the FCN model includes convolution layers, pooling layers, and activation layers. The convolution layer, the pooling layer, and the activation layer perform the convolution processing, the pooling processing, and the activation processing, respectively.

In S1401, a variable L is initialized. The variable L represents an index of a layer to be processed.

In S1402, the type of the layer is determined. If the type of the layer is a convolution layer or a pooling layer, the processing proceeds to S1403, and if the type of the layer is an activation layer, the processing proceeds to S1406.

In S1403, the type of the layer is converted. If the L-th layer is a convolution layer, the L-th layer is converted into a dilated convolution layer, and if the L-th layer is a pooling layer, the L-th layer is converted into a dilated Pooing layer. Parameters such as the size of the kernel, the number of types of kernels, the coefficients of the kernels, and the pooling size of the dilated convolution layer and the dilated pooling layer after the conversion are the same as setting values of the convolution layer and the pooling layer before the conversion.

In S1404, the dilate amount of the L-th layer is determined. An example of a procedure for determining the dilate amounts Dy in the vertical direction will be described. The dilate amount Dx in the horizontal direction is also determined in a similar manner by replacing the subscript from y to x. First, a product Psy of stride amounts Sy in the vertical direction from a 0th layer to a (L-1) th layer of the input FCN model is calculated. Next, a greatest common divisor GCy of the product Psy of the stride amount Sy and a pitch Ky in the vertical direction of the region division result are obtained. The product Psy of the stride amounts Sy and the greatest common divisor GCy are substituted into the following equation, and the dilate amount Dy in the vertical direction of the L-th layer is determined.

[Equation 8]

$$Dy = PSy/GCy$$

Further, the pitch Ky is updated by the following equation.

[Equation 9]

$$Ky = Ky/GCy$$

In S1405, the stride amount of the L-th layer is determined. An example of a determination procedure of the stride amount Sy in the vertical direction will be described. The stride amount Sx in the horizontal direction is also determined in a similar manner by replacing the subscript from y to x. Then, the greatest common divisor GCy calculated in 1404 is substituted into the following equation, and the stride amount Sy of the L-th layer in the vertical direction is determined.

[Equation 10]

$$Sy = GCy$$

In S1406, the variable L is incremented. That is, the processing target is switched to the next layer.

In S1407, it is determined whether the conversion processing for all layers is completed. For example, if the variable L is equal to or greater than the number of layers constructing the FCN model, it is determined that the processing is completed, otherwise, it is determined that the processing is not completed. If the processing is completed, the processing flow ends, and if the processing is not completed, the processing proceeds to S1402.

With the above processing flow, the FCN model can be converted into a DFCN model capable of obtaining a high-resolution region division result at high speed.

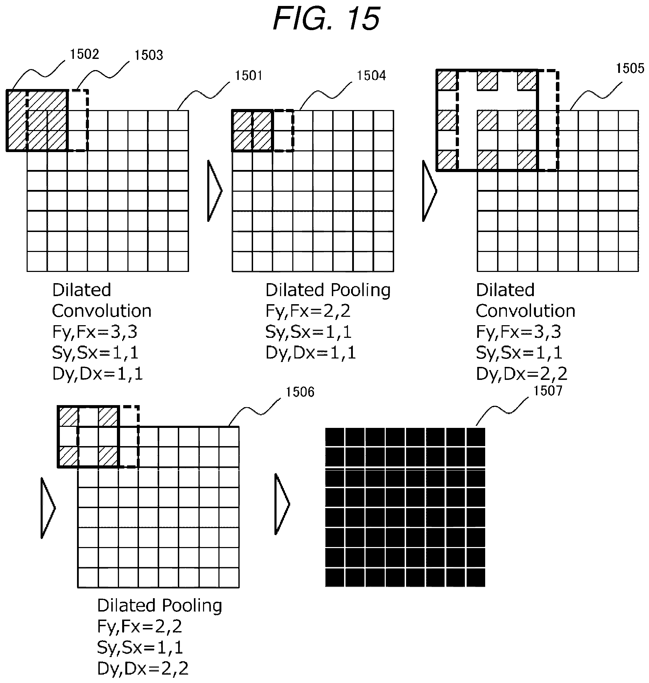

FIG. 15 shows an example of the region segmenting processing by the DFCN. It is assumed that an input image 1501 is an image input to the DFCN and has a width and a height of 8 pixels. A first kernel 1502 indicates a first processing target region in each layer. A shaded part represents a processing target location. A second kernel 1503 indicates a second processing target region in each layer.

A first layer output data 1504 is a result of applying the dilated convolution processing to the input image 1501, and a second layer output data 1505 is a result of applying the dilated pooling processing to the first layer output data 1504. A third layer output data 1506 is a result of applying the dilated convolution processing to the second layer output data 1505, and a fourth layer output data 1507 is a result of applying the dilated pooling processing to the third layer output data 1506. Since the resolution is not reduced by the processing of each layer, the fourth layer output data 1507 becomes a region segmentation result having the same resolution as that of the input image 1501.

In order to obtain a high-resolution region segmentation result by the FCN, it is necessary to perform the region segmentation processing for each image of different acquisition positions as shown in FIG. 10, a part of the processing is overlapped, and thereby the processing time is increased. In the region segmentation processing by DFCN, since the overlapping processing in the high-resolution FCN is completed at once, a high-resolution region segmentation result can be obtained at high speed.

Although the FCN model is converted into the DFCN model in FIG. 14, the DFCN model may be constructed from the beginning. A DNN-based image classifier that identifies an input image of a certain size may be converted into FCN and further converted into DFCN. Machine learning for region segmentation may be performed by a model of a DNN-based image classifier, FCN, and DFCN.

Second Embodiment

The first embodiment describes that the type of the container 112 is determined based on the edge region 402 of the container 112 calculated from the upper image 212. A determination result output by the image processing apparatus 101 is not limited to the type of the container 112. The present embodiment describes that an amount of the collection target 113 is determined based on the edge region 402 of the container 112 and the upper surface region 404 of the collection target 113 calculated from the upper image 212. A hardware configuration and a functional block diagram of the present embodiment are the same as those of the first embodiment, and thus the description thereof will be omitted.

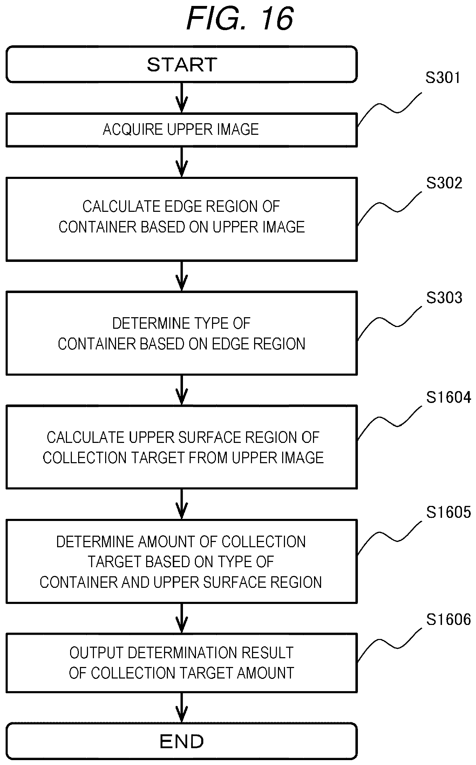

An example of a processing flow of the present embodiment will be described with reference to FIG. 16. S301 to S303 are the same processing as those in the first embodiment, and thus the description thereof will be omitted, and S1604 to S1606 after S303 will be described.

(S1604)

The region calculation unit 202 calculates the upper surface region 404 of the collection target 113 from the upper image 212 acquired in S301. In the calculation of the upper surface region 404, similar to S302, threshold processing using the luminance value of the upper image 212, region segmentation processing using FCN or DFCN, or the like is executed.

(S1605)

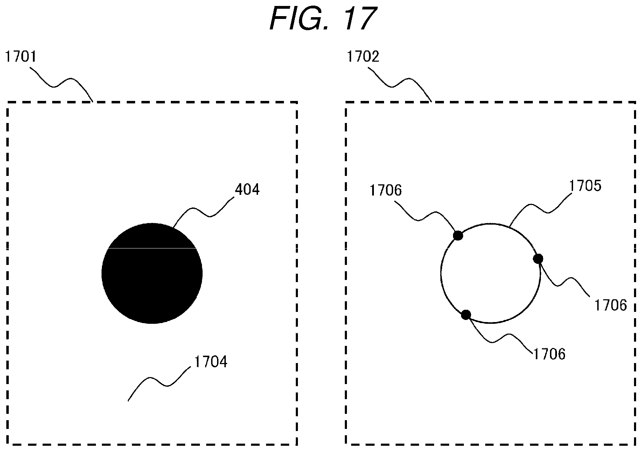

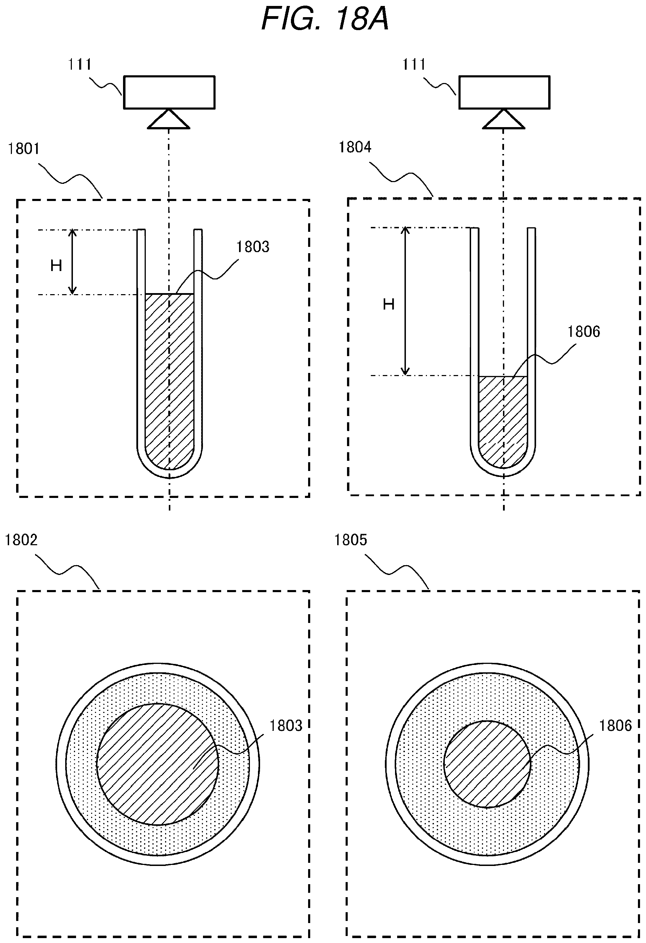

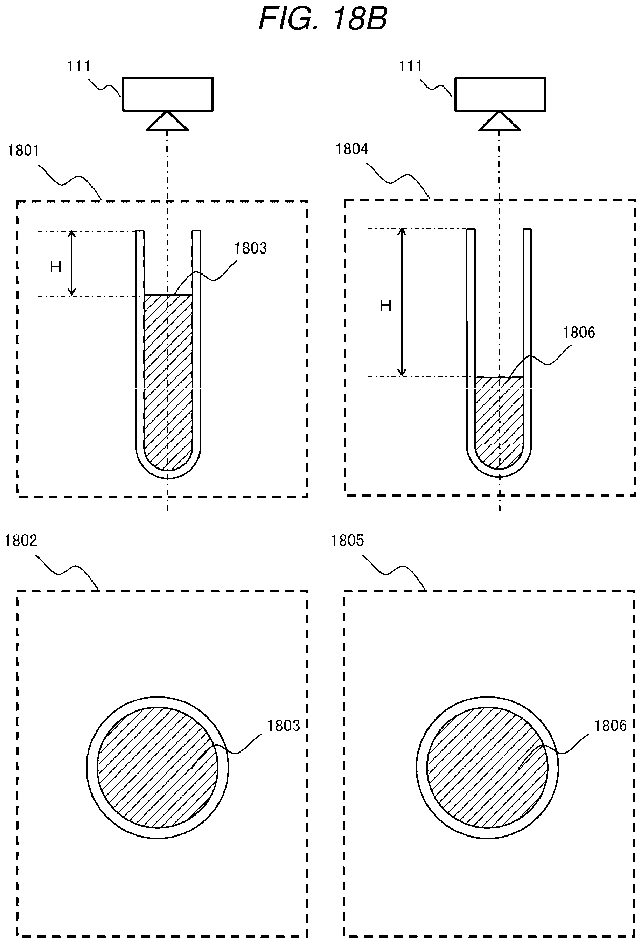

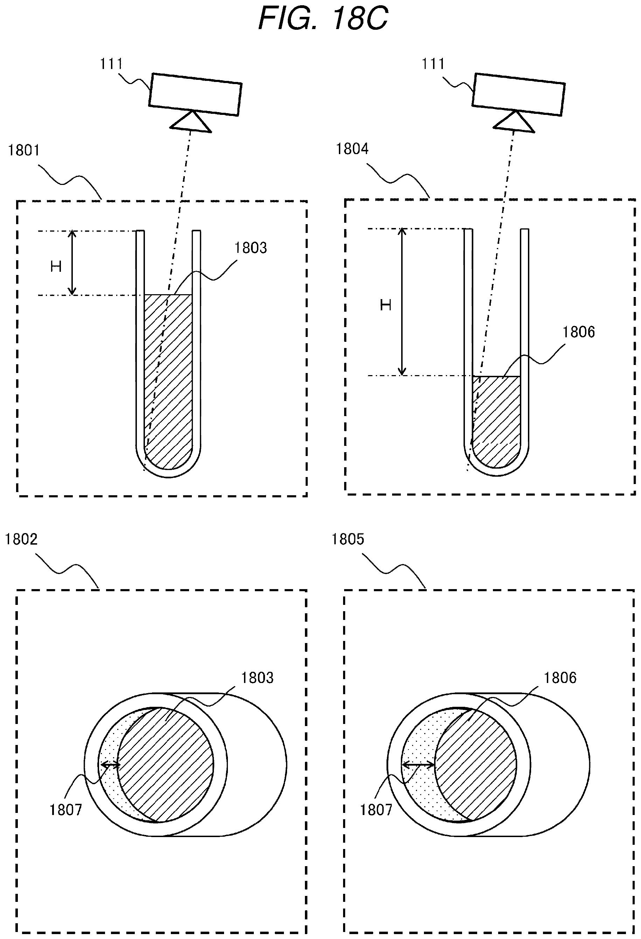

The state determination unit 203 determines the amount of the collection target 113 based on the edge region 402 calculated in S302 and the upper surface region 404 calculated in S1604. The step will be described with reference to FIGS. 17 and 18. First, the upper surface region 404 will be described with reference to FIG. 17.

The state determination unit 203 creates a binarized image 1701 of the upper surface of the collection target 113 based on the calculation result in S1604. The upper surface binarized image 1701 includes the upper surface region 404 and another region 1704 other than the upper surface region 404. For example, a luminance value of 0 is given to the upper surface region 404, and a luminance value of 255 is given to the other region 1704.

The state determination unit 203 extracts a boundary of the upper surface region 404 by applying a Sobel filter or the like to the binarized image 1701 of the upper surface, and creates a boundary image 1702 of the upper surface. The upper surface boundary image 1702 includes an upper surface boundary 1705. The state determination unit 203 performs elliptical fitting on at least three extraction points 1706 arbitrarily extracted from the upper surface boundary 1705, and calculates a long diameter and a short diameter of the upper surface region 404. Since the short diameter of the upper surface region 404 is less likely to change than the long diameter even when the container 112 is inclined, the short diameter is used in the subsequent processing.

The determination of the amount of the collection target 113 will be described with reference to FIGS. 18A to 18C. A case where a lens of the imaging unit 111 is non-telecentric and a case where the lens of the imaging unit 111 is telecentric will be described. When the lens is non-telecentric, a size of an imaged object decreases as a distance from the imaging unit 111 to the imaged object increases, whereas when the lens is telecentric, the size of the imaged object remains the same regardless of the distance from the imaging unit 111 to the imaging object.

A case of a non-telecentric lens will be described with reference to FIG. 18A. FIG. 18A is a side view or an upper image when an amount of the collection target 113 is large or small, that is, a side view 1801 in a case of a large amount, an upper image 1802 in a case of a large amount, a side view 1804 in a case of a small amount and an upper image 1805 in a case of a small amount are shown. As can be seen from the comparison between the upper image 1802 in the case of the large amount and the upper image 1805 in the case of the small amount, an area of the upper surface 1803 in the case of the large amount is larger than an area of the upper surface 1806 in the case of the small amount. Therefore, the state determination unit 203 determines the amount of the collection target 113 based on the short diameter of the upper surface region 404.

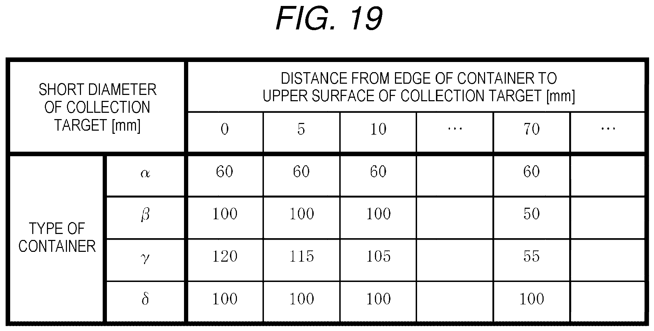

Specifically, first, the short diameter of the upper surface region 404 is collated with a table shown in FIG. 19, and a distance H from the edge 213 of the container 112 to the upper surface 215 of the collection target 113 is obtained. The table of FIG. 19 is created in advance by measuring the short diameter and the distance H of the upper surface region 404 when different amounts of collection targets 113 are stored in a plurality of types of containers 112, and is stored in the memory 104 or the like. Next, the type of the container 112 determined based on the edge region 402 is collated with the table of FIG. 6B, and the length G and the inner diameter D of the container 112 are obtained. Then, the distance H, the length G, and the inner diameter D are substituted into the following equation, and an amount Q of the collection target 113 is obtained.

[Equation 11]

$$Q = \pi * \left(\frac{D}{2}\right)^2 * (G - H)$$

Equation 11 is an expression in a case where the inner diameter D of the container 112 is constant regardless of the distance H. When the container 112 has a tapered shape or the like and the inner diameter D of the container 112 changes depending on the distance H, a relationship between the distance H and the inner diameter D is stored in advance in the memory 104 or the like, and is referred to as necessary, whereby the amount Q of the collection target 113 is obtained using Equation 11.

A case of a telecentric lens will be described with reference to FIG. 18B. Similar to FIG. 18A, FIG. 18B shows the side view 1801 in the case of a large amount, the upper image 1802 in the case of the large amount, the side view 1804 in the case of the small amount, and the upper image 1805 in the case of the small amount. As can be seen from the comparison between the upper image 1802 in the case of the large amount and the upper image 1805 in the case of the small amount, in the case of the telecentric lens, areas of the upper surface 1803 in the case of the large amount are equal to areas of the upper surface 1806 in the case of the small amount, and the amount of the collection target 113 cannot be determined based on the short diameter of the upper surface region 404.

Therefore, in the case of the telecentric lens, the amount of the collection target 113 is determined based on the upper image captured by inclining the imaging unit 111 as shown in FIG. 18C. That is, as can be seen from a comparison between the upper image 1802 in the case of the large amount and the upper image 1805 in the case of the small amount, since a distance 1807 from the edge to the upper surface is shorter in the case of the large amount of the collection target 113 than in the case of the small amount of the collection target 113, the amount of the collection target 113 is determined based on the distance 1807 from the edge to the upper surface. Specifically, when the distance 1807 from the edge to the upper surface is H' and an inclination angle of the imaging unit 111 with respect to the vertical direction is $\Psi$, the distance H from the edge 213 of the container 112 to the upper surface 215 of the collection target 113 is obtained by the following equation, and the amount Q of the collection target 113 is obtained by Equation 11.

[Equation 12]

$$H = \frac{H'}{\sin \psi}$$

When the container 112 has a tapered shape or the like, similar to the case of the non-telecentric lens, a relationship between the distance H and the inner diameter D stored in advance in the memory 104 or the like is referred to as necessary, and the amount Q of the collection target 113 is obtained using Equation 11.

(S1606)

The output unit 105 outputs the amount of the collection target 113 determined in S1605. The result of the determination is transmitted to, for example, the control unit 121, and the control unit 121 controls the collection unit 122 based on the result of the determination. For example, when it is determined that the amount of the collection target 113 does not reach a predetermined amount, the control unit 121 may stop the collection unit 122. The output unit 105 may output the result of the determination to a display or a printer to present the amount of the collection target 113 to the operator, or may display a message indicating that the amount of the collection target 113 does not reached a predetermined amount when the amount of the collection target 113 does not reached the predetermined amount.

According to the processing flow described above, the image processing apparatus 101 can determine the amount of the collection target 113, which is one of the states of the collection target 113, based on the upper image 212 acquired by the single imaging unit 111. The automatic analysis system 100 can appropriately control the collection unit 122 according to the determination result by the image processing apparatus 101, and thus can perform more accurate analysis without reducing an inspection efficiency.

[Third Embodiment]

In the first embodiment, the determination of the type of the container 112 has been described, and in the second embodiment, the determination of the amount of the collection target 113 has been described. The present embodiment describes that an inclination of the container 112 is determined based on the edge region 402 of the container 112 and the upper surface region 404 of the collection target 113 calculated from the upper image 212. A hardware configuration and a functional block diagram of the present embodiment are the same as those of the first embodiment, and thus the description thereof will be omitted.

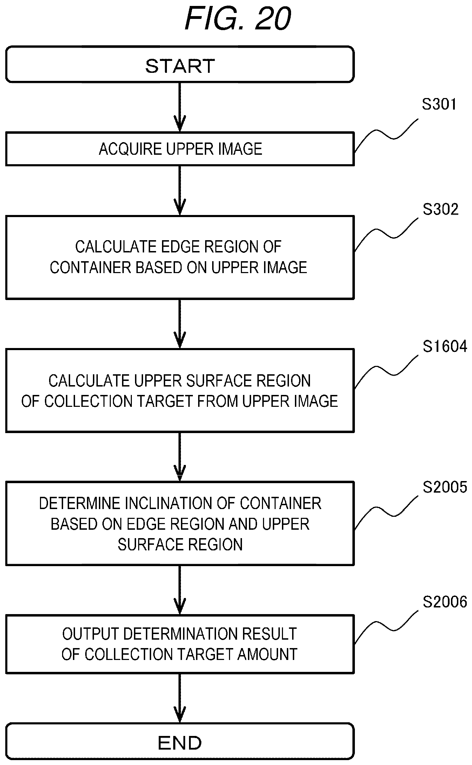

An example of a processing flow of the present embodiment will be described with reference to FIG. 20. S301 to S302 are the same processing as those in the first embodiment, S1604 is the same processing as that in the second embodiment and thereby the description is omitted, and steps S2005 to S2006 subsequent to step S1604 will be described.

(S2005)

The state determination unit 203 determines the inclination of the container 112 based on the edge region 402 calculated in S302 and the upper surface region 404 calculated in S1604. In the determination of the inclination of the container 112, the presence or absence of the inclination or the inclination angle is determined.

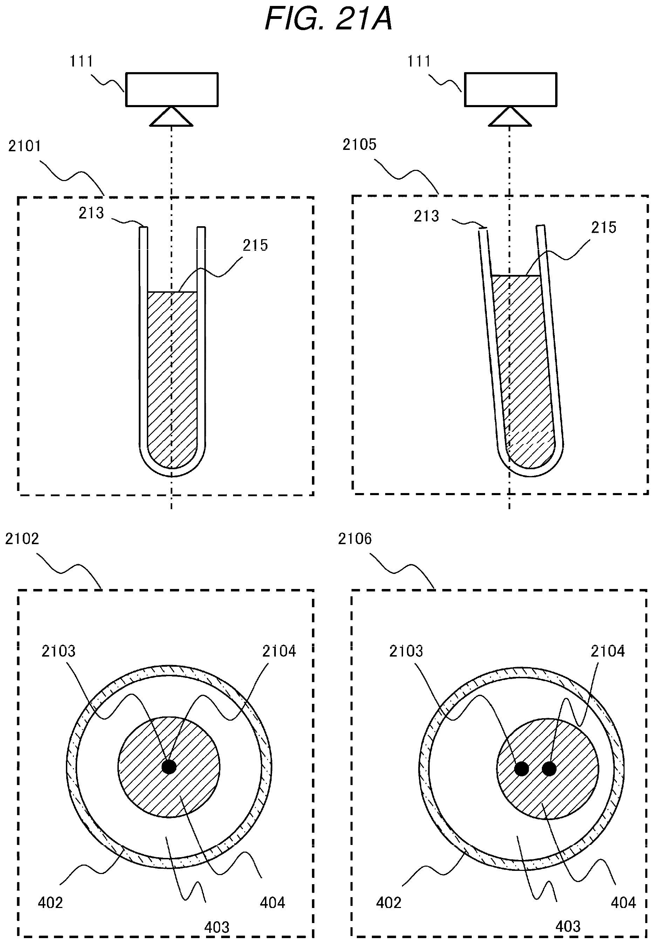

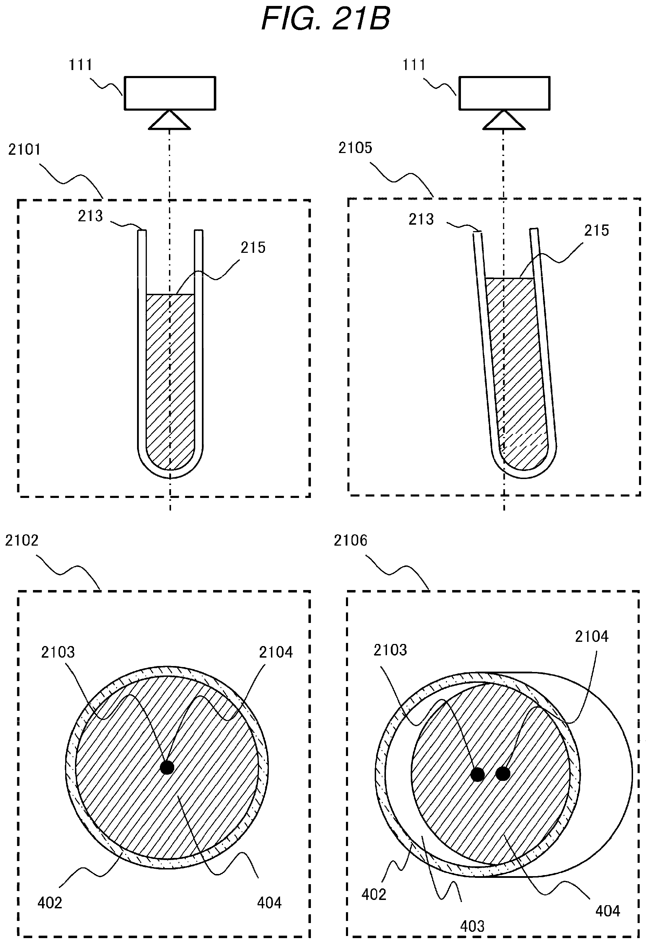

The determination of the presence or absence of the inclination of the container 112 will be described with reference to FIG. 21. In the present embodiment, a case where the lens of the imaging unit 111 is non-telecentric and a case where the lens of the imaging unit 111 is telecentric will be described.

The case of the non-telecentric lens will be described with reference to FIG. 21A. FIG. 21A shows side views or upper images of the container 112 when the container 112 is upright and inclined, that is, a side view 2101 when the container 112 is upright and an upper image 2102 when the container 112 is upright, a side view 2105 when the container 112 is inclined and an upper image 2106 when the container 112 is inclined. In the upright upper image 2102 and the inclined upper image 2106, a center 2103 of the edge region 402 and a center 2104 of the upper surface region 404 are obtained based on the edge region 402 calculated in S302 and the upper surface region 404 calculated in S1604.

As can be seen from the comparison between the upright upper image 2102 and the inclined upper image 2106, the center 2103 of the edge region 402 and the center 2104 of the upper surface region 404 coincide with each other in an upright state, whereas the center 2103 and the center 2104 do not coincide with each other in an inclined state, and a distance between the center 2103 and the center 2104 increases as the inclination increases. Therefore, the state determination unit 203 determines the presence or absence of the inclination of the container 112 based on the distance between the center 2103 of the edge region 402 and the center 2104 of the upper surface region 404. That is, when the distance between the center 2103 of the edge region 402 and the center 2104 of the upper surface region 404 is equal to or greater than a predetermined threshold value, it is determined that the container 112 is inclined, and when the distance is less than the threshold value, it is determined that the container 112 is not inclined.

The case of the telecentric lens will be described with reference to FIG. 21B. Similar to in FIG. 21A, FIG. 21B shows the side view 2101 when the container 112 is upright and the upper image 2102 when the container 112 is upright, the side view 2105 when the container 112 is inclined and the upper image 2106 when the container 112 is inclined. Similar to the case of the non-telecentric lens, the center 2103 of the edge region 402 and the center 2104 of the upper surface region 404 are obtained, and also in the case of the telecentric lens, the presence or absence of inclination of the container 112 is determined based on the distance between the center 2103 and the center 2104. The determination of the presence or absence of the inclination of the container 112 based on the distance between the center 2103 and the center 2104 can also be performed when the container 112 has a tapered shape.

In addition to the distance between the center 2103 of the edge region 402 and the center 2104 of the upper surface region 404, the presence or absence of the inclination of the container 112 may be determined based on the inner wall region 403 or the like of the container 112. For example, since the shape of the inner wall region 403 changes when the container 112 is inclined compared with when the container 112 is upright, the presence or absence of inclination of the container 112 may be determined using a width or area of the inner wall region 403. Not only the inner wall region 403 but also an outer wall region of the container 112 may be used to determine the presence or absence of the inclination of the container 112.

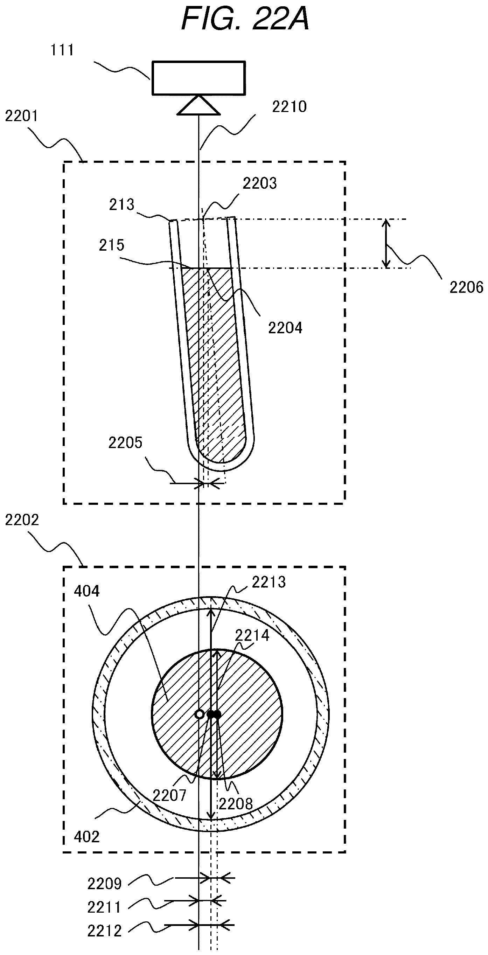

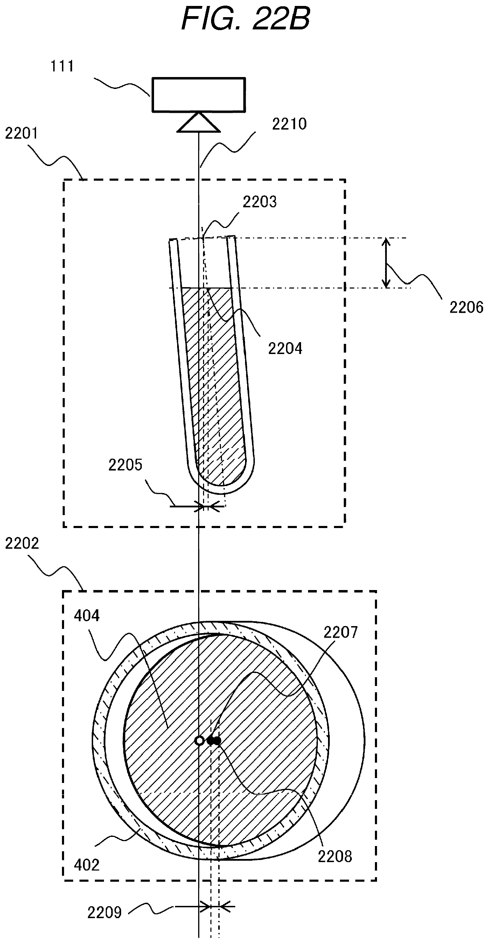

The determination of an angle of the inclination of the container 112 will be described with reference to FIGS. 22A and 22B. In the present embodiment, the case where the lens of the imaging unit 111 is non-telecentric and the case where the lens of the imaging unit 111 is telecentric will be described.

The case of the non-telecentric lens will be described with reference to FIG. 22A. FIG. 22A shows a side view 2201 when the container 112 is inclined and an upper image 2202 when the container 112 is inclined. In order to obtain the inclination angle of the container 112 in the side view 2201 at the time of inclination, a distance in the horizontal direction and a distance in the vertical direction between the center 2203 of the edge 213 of the container 112 and the center 2204 of the upper surface 215 of the collection target 113 may be calculated. That is, an inclination angle $\theta$ of the container 112 with respect to the vertical direction is obtained from a center-to-center horizontal distance 2205, which is a distance in the horizontal direction between the center 2203 and the center 2204, and a center-to-center vertical distance 2206, which is a distance in the vertical direction.

[Equation 13]

$$\theta = \tan^{-1} \frac{Sh}{H}$$

Here, Sh is the center-to-center horizontal distance 2205, and H is the center-to-center vertical distance 2206.

However, since the image captured by the imaging unit 111 is the upper image 2202 at the time of inclination, it is necessary to acquire the center-to-center horizontal distance 2205 and the center-to-center vertical distance 2206 from the upper image 2202 at the time of inclination in order to obtain the inclination angle $\theta$ of the container 112 using Equation 13. First, the center-to-center vertical distance 2206, that is, the distance H from the edge 213 of the container 112 to the upper surface 215 of the collection target 113 is obtained by comparing the short diameter of the upper surface region 404 with the table shown in FIG. 19, as described in the second embodiment.

Next, the center-to-center horizontal distance 2205 is obtained from the upper image 2202 at the time of inclination. In the case of the non-telecentric lens, since the size of the imaged object decreases as a distance from the imaging unit 111 to the imaged object increases, it is preferable that a length of the collection target 113 on the upper surface 215 is corrected to the length of the edge 213 of the container 112 and handled. Specifically, it is preferable that a region center-to-center distance 2209, which is the distance between a center 2207 of the edge region 402 and a center 2208 of the upper surface region 404 in the upper image 2202 at the time of inclination, is corrected based on a ratio between the short diameter of the upper surface region 404 and the inner diameter of the edge region 402. For example, Sh, which is the center-to-center horizontal distance 2205, is calculated by the following equation.

[Equation 14]

$$Sh = \left\| F1 * \frac{B0}{B1} - F0 \right\|$$

Here, F1 is a first center-to-center distance 2212 that is a distance from a lens center 2210 to the center 2208 of the upper surface region 404, and F0 is a second center-to-center distance 2211 that is a distance from the lens center 2210 to the center 2207 of the edge region 402. B0 is an inner diameter 2213 of the edge region 402, and B1 is a short diameter 2214 of the upper surface region 404. According to Equation 14, after the second center-to-center distance 2211, which is the length of the collection target 113 on the upper surface 215, is corrected to the length of the container 112 on the edge 213, a difference between the second center-to-center distance 2211 and the first center-to-center distance 2212 is calculated, and an absolute value thereof is calculated as Sh, which is the center-to-center horizontal distance 2205. That is, the size of the imaged object, which changes according to the distance from the imaging unit 111 to the imaged object, is corrected, and Sh, which is the center-to-center horizontal distance 2205, is calculated.

By substituting the calculated Sh and the previously obtained distance H into Expression 13, the inclination angle θ of the container 112 is obtained.

The case of the telecentric lens will be described with reference to FIG. 22B. In FIG. 22B, similar to FIG. 22A, the side view 2201 when the container 112 is inclined and the upper image 2202 when the container 112 is inclined are shown. In the case of the telecentric lens, the center-to-center horizontal distance 2205 is obtained as the region center-to-center distance 2209 that is the distance between the center 2207 of the edge region 402 and the center 2208 of the upper surface region 404 in the upper image 2202 at the time of inclination. Since the center-to-center vertical distance 2206 is obtained in a similar manner as in the second embodiment, the center-to-center horizontal distance 2205 and the center-to-center vertical distance 2206 are substituted into Equation 13 to obtain the inclination angle θ of the container 112.

The inclination angle θ of the container 112 may be an inclination angle of the imaging unit 111 when the imaging unit 111 is inclined as shown in FIG. 18C and the inner wall region 403 of the container 112 is no longer included in the upper image.

(S2006)

The output unit 105 outputs the inclination of the container 112 determined in S2005. The result of the determination is transmitted to, for example, the control unit 121, and the control unit 121 controls the collection unit 122 based on the result of the determination. For example, when it is determined that the container 112 is inclined or the inclination angle of the container 112 exceeds a predetermined threshold value, the control unit 121 may stop the collection unit 122. Alternatively, the position and inclination of the collection unit 122 may be adjusted according to the inclination angle of the container 112.

The output unit 105 may output the determination result to a display or a printer and present to the operator whether the container 112 is inclined and the inclination angle, and a message or the like calling attention may be displayed when a ratio of the number of inclined containers 112 exceeds a predetermined threshold value. Further, the output unit 105 may output the inclination angle of the container 112 to another image processing apparatus, and may output an image on which a correction processing according to the type of the container 112 is performed together with the inclination angle of the container 112. The correction processing according to the inclination angle of the container 112 is, for example, processing of correcting deformation of the image due to the inclination of the container 112 by converting coordinates of the image according to the inclination angle of the container 112.

According to the processing flow described above, the image processing apparatus 101 can determine the inclination of the container 112, which is one of the states of the container 112, based on the upper image 212 acquired by the single imaging unit 111. The automatic analysis system 100 can appropriately control the collection unit 122 according to the determination result by the image processing apparatus 101, and thus can perform more accurate analysis without reducing an inspection efficiency.

[Fourth Embodiment]

The first to third embodiment describes that the states of the container 112 and the collection target are determined based on the edge region 402 of the container 112 and the upper surface region 404 of the collection target 113 calculated from the upper image 212. In order for the image processing apparatus 101 to accurately perform region segmentation processing on the upper image 212 using FCN, DFCN, or the like, an appropriate teacher signal used for machine learning is required. In the embodiment, it will be described that a teacher signal for machine learning is efficiently created. A hardware configuration of the present embodiment is the same as that of the first embodiment, and thus the description thereof will be omitted.

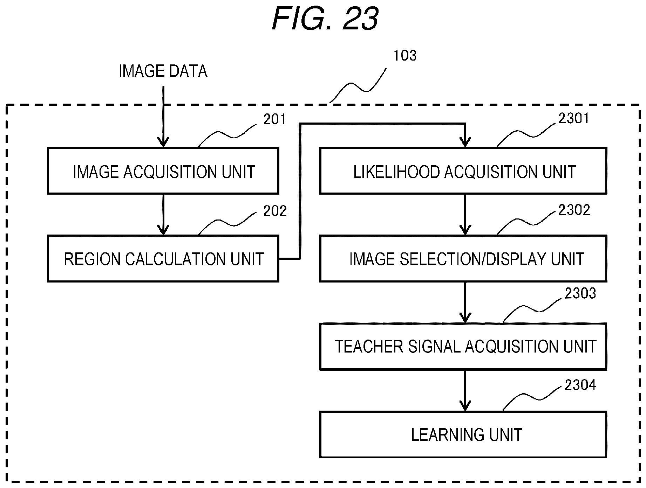

An example of a functional block diagram of the present embodiment will be described with reference to FIG. 23. The functions may be configured with dedicated hardware, or may be configured with software operating on the calculation unit 103. The embodiment includes the image acquisition unit 201 and the region calculation unit 202, as well as a likelihood acquisition unit 2301, an image selection/display unit 2302, a teacher signal acquisition unit 2303, and a learning unit 2304. Hereinafter, each unit other than the image acquisition unit 201 and the region calculation unit 202 described in the first embodiment will be described.

The likelihood acquisition unit 2301 acquires the likelihood that whether each pixel in each region calculated by the region calculation unit 202 is, for example, the edge region 402, the inner wall region 403, the upper surface region 404, or the like. The likelihood is normalized such that a sum is 1.0 for each pixel, and a maximum likelihood in each pixel is called the maximum likelihood. A pixel with a large maximum likelihood has a high degree of certainty in a corresponding region, and a pixel with a small maximum likelihood has a low certainty degree.

The image selection/display unit 2302 selects an image to be used for applying the teacher signal based on, for example, the maximum likelihood of each pixel in the image, and displays the selected image on a display or the like.

The teacher signal acquisition unit 2303 acquires a teacher signal from a teacher image that is an image including the teacher signal. The teacher signal is assigned by the operation of the operator or the region segmentation processing by another image processing apparatus.

The learning unit 2304 performs machine learning using the teacher signal included in the teacher image, and the region calculation unit 202 calculates or updates parameters such as coefficients used in the region segmentation processing.

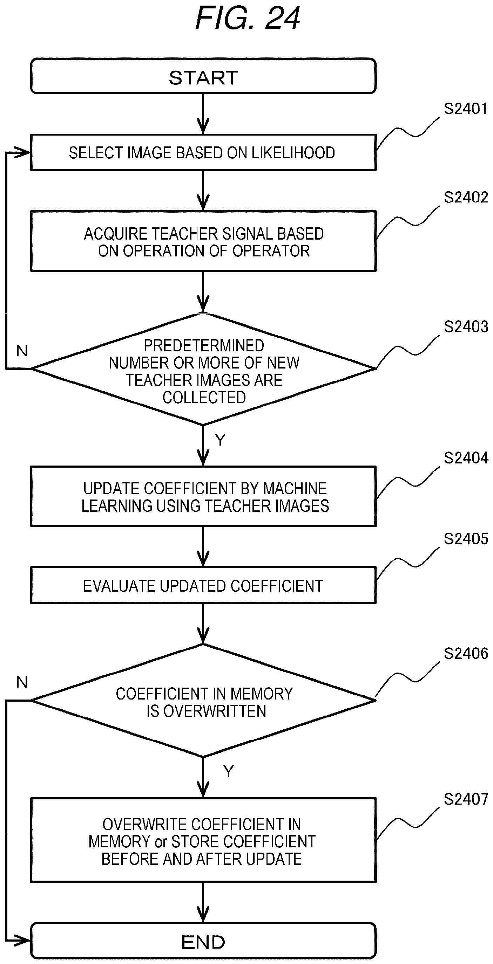

An example of a processing flow of the present embodiment will be described with reference to FIG. 24. In the present embodiment, an image having a relatively low certainty degree of each segmented region is selected and displayed, and the machine learning is performed using the teacher signal assigned by the operator to the displayed image.

(S2401)

The image selection/display unit 2302 selects an image for assigning a teacher signal from a group of images recorded on a recording device or the like based on the likelihood of each pixel in the image, and displays the image on a display or the like. For example, an image in which the average value of the maximum likelihood of each pixel is less than a predetermined threshold value, that is, an image in which the certainty degree of each region is relatively low is selected.

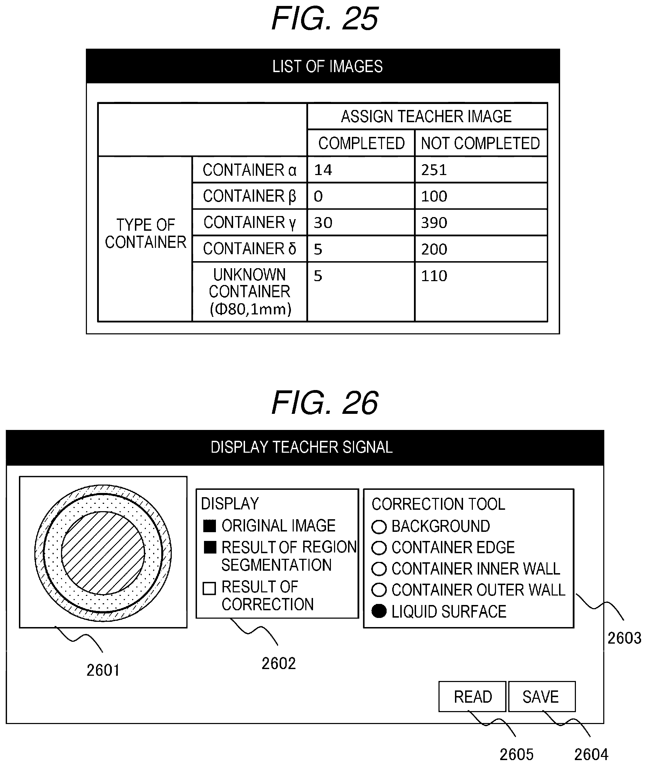

An example of the screen displayed in this step will be described with reference to FIG. 25. The screen shown in FIG. 25 is a list of images indicating the number of images to which the teacher signal has been assigned and the number of images to which no teacher signal has been assigned among a plurality of images selected in this step for each type of the container 112. When an arbitrary cell in the list of images, for example, a cell in which 100, which is a not-completed container β, is displayed, is selected by a mouse cursor or the like, an image including the container β, which is an image corresponding to the cell, may be displayed.

The operator can check the number of images to which the teacher signal is assigned and the number of images to which no teacher signal is assigned from the screen for each type of the container 112, and can determine an image including a predetermined type of container 112 to which the teacher signal should be assigned, thereby improving the efficiency of the operation of assigning the teacher signal. The displayed list of images is not limited to a list classified for each type of the container 112.

(S2402)

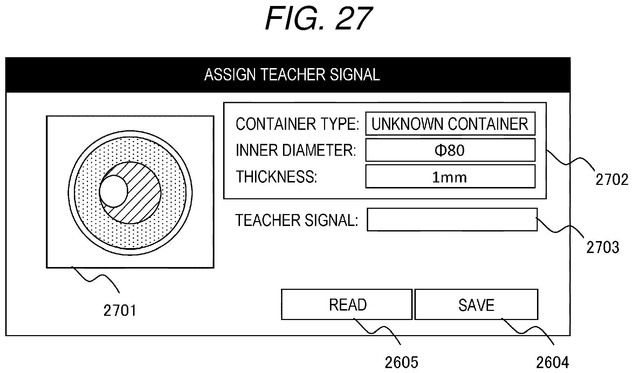

The teacher signal acquisition unit 2303 acquires the teacher signal for the image displayed in S2401 based on the operation of the operator. An example of the screen used in this step will be described with reference to FIGS. 26 and 27.

The screen shown in FIG. 26 is an example of an operation screen for designating a region to which the teacher signal is to be assigned, and includes an image display portion 2601, an image selection portion 2602, a correction tool 2603, a save button 2604, and a read button 2605.

The image display portion 2601 displays an image corresponding to the selection by the image selection portion 2602. The image selection portion 2602 selects a condition of an image displayed on the image display portion 2601. In FIG. 26, an original image and a region segmentation result are selected by the image selection portion 2602, and the result of the region segmentation of the original image by the region calculation unit 202 is displayed on the image display portion 2601.

In the correction tool 2603, a region in which the teacher signal is desired to be corrected is selected. For example, when an arbitrary pixel of the image displayed in the image display portion 2601 is selected in a state where the liquid surface, that is, the upper surface when the collection target 113 is liquid is selected by the correction tool 2603, a teacher signal of the upper surface region 404 is assigned to the selected pixel. The selection of pixel in the image displayed in the image display portion 2601 is performed by a mouse or the like.

The save button 2604 is a button to be pressed when a corrected teacher signal is saved. That is, when the save button 2604 is pressed, the teacher signal acquisition unit 2303 acquires the assigned teacher signal via the correction tool 2603. The original teacher signal is acquired for the pixel that has not been corrected by the correction tool 2603.

The read button 2605 is a button to be pressed when another image is selected. When the read button 2605 is pressed, for example, the image displayed in the image display portion 2601 may be updated, or the screen shown in FIG. 25 may be displayed.

The screen shown in FIG. 27 is an example of an operation screen for assigning a teacher signal, and includes an upper image display portion 2701, a container type display portion 2702, a teacher signal input portion 2703, the save button 2604, and the read button 2605. The save button 2604 and the read button 2605 have the same functions as those of the screen of FIG. 26, and thus the description thereof is omitted.

The upper image 212, which is an image captured by the imaging unit 111, is displayed in the upper image display portion 2701. FIG. 27 shows a state in which the mixture 216 such as bubbles or lipids floats on the upper surface 215 of the collection target 113.

In the container type display portion 2702, the type of the container 112 determined based on the region calculated by the region calculation unit 202 is displayed together with the inner diameter and the thickness. A length of the container 112, a printing content, a printing position, the presence or absence of a screw around the edge, a rib on the bottom surface, and the like may be displayed. Various kinds of information displayed in the container type display portion 2702 may be modified by the operator.

In the teacher signal input portion 2703, the teacher signal assigned to an arbitrary region of the image displayed on the upper image display portion 2701 is input. For example, a teacher signal corresponding to the upper surface region 404 is input to bubbles, lipids, or the like in the image displayed in the upper image display portion 2701. The teacher signal input portion 2703 is not limited to a text box shown in FIG. 27, and may have a format in which options such as radio buttons and pull-down menus are presented.

The screens as shown in FIGS. 26 and 27 serve as work assistance for the operator to assign a teacher signal, and thereby it is possible to improve the efficiency of the work of assigning a teacher signal.

(S2403)

It is determined whether a predetermined number or more of new teacher images are collected. If the predetermined number or more of teacher images are collected, the processing proceeds to S2404, and if the predetermined number or more of teacher images are not collected, the processing returns to S2401.

(S2404)

The learning unit 2304 updates the coefficient by machine learning using the predetermined number or more of teacher images. For the update of the coefficient, for example, a stochastic gradient descent method is used. As an initial value of the coefficient, the coefficient stored in the memory 104 may be used, or a random number may be used.

(S2405)

The learning unit 2304 evaluates the updated coefficient. For example, an image group with correct answer information is prepared in advance for evaluation of the coefficient, and a region segmentation processing using the coefficient updated in S2404 and the coefficient before update is performed on the image group, and the coefficient after update is evaluated based on whether a correct answer rate increases.

(S2406)

It is determined whether a coefficient in the memory 104 is overwritten. An evaluation result in S2405 may be used to determine whether the information is overwritten. If the coefficient is overwritten, the processing proceeds to S2407, and if the coefficient is not overwritten, the processing flow ends.

(S2407)

The learning unit 2304 overwrites the coefficient in the memory 104 and stores the coefficients before and after the update.

According to the processing flow described above, an appropriate teacher signal is efficiently assigned to an image having a relatively low certainty degree of a region segmentation result, and the image processing apparatus 101 can efficiently perform appropriate machine learning since an appropriate teacher signal is efficiently assigned. An appropriate teacher signal to air bubbles, foreign matter, and the like can be applied. Since the accuracy of the region segmentation processing can be improved by appropriate machine learning, the image processing apparatus 101 can more accurately determine the state of the container 112 or the collection target 113, and the automatic analysis system 100 can perform analysis more accurately.

A plurality of embodiments of the invention have been described above. The invention is not limited to these embodiments, and includes various modifications. For example, the embodiments described above are described in detail for easy understanding of the invention, and the invention is not limited to those including all the configurations described above. A part of the configuration of one embodiment can be replaced with the configuration of another embodiment. Further, a configuration of another embodiment can be added to the configuration of one embodiment. A part of the configuration of one embodiment may be added, deleted, or replaced with another configuration.

**Claims**

1.  An image processing apparatus comprising:

    an image acquisition unit configured to acquire an upper image that is an image obtained by capturing an image of a container storing a collection target including a sample, a reagent, and a reaction solution from above;
    a region calculation unit configured to calculate an edge region of the container or an upper surface region of the collection target from the upper image; and
    a state determination unit configured to determine a state of the container or a state of the collection target based on the edge region or the upper surface region.

2.  The image processing apparatus according to claim 1, wherein

    the region calculation unit calculates the edge region, and
    the state determination unit determines a type of the container based on an outer diameter and an inner diameter of the edge region.

3.  The image processing apparatus according to claim 2, wherein

    the region calculation unit further calculates the upper surface region, and
    the state determination unit determines an amount of the collection target based on the type of the container and a size of the upper surface region or a distance from the upper surface region to the edge region.

4.  The image processing apparatus according to claim 1, wherein

    the region calculation unit calculates the edge region and the upper surface region, and
    the state determination unit determines whether the container is inclined based on a degree of coincidence between a center of the edge region and a center of the upper surface region.

5.  The image processing apparatus according to claim 3, wherein
    the state determination unit determines an inclination angle of the container based on a height of the collection target obtained using the type of the container and the size of the upper surface region, and a center distance that is a distance between a center of the edge region and a center of the upper surface region.

6.  The image processing apparatus according to claim 5, wherein
    when the upper image is captured using a non-telecentric lens, the state determination unit corrects the center distance based on a ratio between a short diameter of the upper surface region and the inner diameter of the edge region.

7.  The image processing apparatus according to claim 1, further comprising:

    a display unit configured to display a selected image; and
    a teacher signal acquisition unit configured to display an operation screen for inputting a teacher signal for the image displayed on the display unit.

8.  The image processing apparatus according to claim 7, wherein
    the display unit further displays a screen indicating the number of selected images.

9.  The image processing apparatus according to claim 1, wherein
    the region calculation unit segments the upper image into a plurality of regions by performing a convolution processing and a pooling processing using a dilate kernel created by inserting a predetermined number of zeros between elements of a kernel, and calculates the edge region or the upper surface region.

10. The image processing apparatus according to claim 9, wherein
    the region calculation unit determines the number of zeros to be inserted into the kernel on a basis of a stride amount that is an amount by which the dilate kernel is shifted when the convolution processing and the pooling processing are executed.

11. An automatic analysis system including the image processing apparatus according to claim 1, the system comprising:

   a collection unit configured to collect the collection target from the container; and
   a control unit configured to control the collection unit based on a determination result of the state determination unit.

12. The automatic analysis system according to claim 11, wherein
   the control unit stops the collection unit when the state determination unit determines that a type of the container is an unknown type, when the state determination unit determines that an amount of the collection target is less than a threshold value, or when the state determination unit determines that an inclination angle of the container is equal to or greater than a threshold value.

13. The automatic analysis system according to claim 11, wherein
   the control unit adjusts a height and an angle of the collection unit in accordance with a type of the container, an amount of the collection target, and an inclination angle of the container determined by the state determination unit.

14. An image processing method comprising:

   an image acquiring step of acquiring an upper image that is an image obtained by capturing an image of a container storing a collection target including a sample, a reagent, and a reaction solution from above;
   a region calculating step of calculating an edge region of the container or an upper surface region of the collection target from the upper image; and
   a state determination step of determining a state of the container or a state of the collection target based on the edge region or the upper surface region.

15. An image processing method for segmenting an image into a plurality of regions, the method comprising:

   creating a dilate kernel by inserting a predetermined number of zeros between elements of a kernel used in a convolution processing and a pooling processing; and
   executing the convolution processing and the pooling processing by using the dilate kernel.

# FIG. 1

## FIG. 2

DETERMINATION RESULT

# FIG. 3

```
┌─────────────────────────────────────┐
│               START                 │
└─────────────────────────────────────┘
                  │
                  ▼
┌─────────────────────────────────────┐      S301
│        ACQUIRE UPPER IMAGE          │
└─────────────────────────────────────┘
                  │
                  ▼
┌─────────────────────────────────────┐      S302
│        CALCULATE EDGE REGION        │
│    OF CONTAINER FROM UPPER IMAGE    │
└─────────────────────────────────────┘
                  │
                  ▼
┌─────────────────────────────────────┐      S303
│          DETERMINE TYPE OF          │
│   CONTAINER BASED ON EDGE REGION    │
└─────────────────────────────────────┘
                  │
                  ▼
┌─────────────────────────────────────┐      S304
│        OUTPUT DETERMINATION         │
│     RESULT OF TYPE OF CONTAINER     │
└─────────────────────────────────────┘
                  │
                  ▼
┌─────────────────────────────────────┐
│                END                  │
└─────────────────────────────────────┘
```

## FIG. 4

# FIG. 5

501

502

402

507

503

506

505

508

507

508

## FIG. 6A

| TYPE OF CONTAINER | | THICKNESS [pix] | | | | |
|---|---|---|---|---|---|---|
| | | 2 | 4 | 6 | 8 | 10 |
| INNER DIAMETER [pix] | 60 | | | | | α |
| | 80 | | | | | |
| | 100 | | β | δ | | |
| | 120 | | | γ | | |

## FIG. 6B

| TYPE OF CONTAINER | THICKNESS [pix] | INNER DIAMETER [pix] | LENGTH [mm] | TAPER |
|---|---|---|---|---|
| α | 10 | 60 | 75 | NO |
| β | 4 | 100 | 100 | YES |
| γ | 6 | 120 | 100 | YES |
| δ | 6 | 100 | 120 | NO |

# FIG. 7

*FIG. 8*

# FIG. 9

Convolution
Fy,Fx=3,3
Sy,Sx=1,1

Pooling
Fy,Fx=2,2
Sy,Sx=2,2

Convolution
Fy,Fx=3,3
Sy,Sx=1,1

Pooling
Fy,Fx=2,2
Sy,Sx=2,2

## FIG. 10

## FIG. 11

| a | b | c |
|---|---|---|
| d | e | f |
| g | h | i |

1101

| a | 0 | b | 0 | c |
|---|---|---|---|---|
| 0 | 0 | 0 | 0 | 0 |
| d | 0 | e | 0 | f |
| 0 | 0 | 0 | 0 | 0 |
| g | 0 | h | 0 | i |

1102

Convolution
$F_y, F_x = 3, 3$
$(D_y, D_x = 1, 1)$

Dilated Convolution
$F_y, F_x = 3, 3$
$D_y, D_x = 2, 2$

## FIG. 12

| 1 | 1 | 1 |
|---|---|---|
| 1 | 1 | 1 |
| 1 | 1 | 1 |

1201

| 1 | 0 | 1 | 0 | 1 |
|---|---|---|---|---|
| 0 | 0 | 0 | 0 | 0 |
| 1 | 0 | 1 | 0 | 1 |
| 0 | 0 | 0 | 0 | 0 |
| 1 | 0 | 1 | 0 | 1 |

1202

Pooling
$F_y, F_x = 3, 3$
$(D_y, D_x = 1, 1)$

Dilated Pooling
$F_y, F_x = 3, 3$
$D_y, D_x = 2, 2$

# FIG. 13

Convolution or Pooling
Fy,Fx=3,3
Sy,Sx=2,2
(Dy,Dx=1,1)

Dilated Convolution or Pooling
Fy,Fx=3,3
Sy,Sx=2,2
Dy,Dx=2,2

## FIG. 14

```
                    START
                      │
                      ▼
                   SET L=0                           S1401
                      │
          ┌───────────┤
          │           ▼
          │    N  ╱L-TH LAYER IS ╲                   S1402
          │  ┌───╲Convolution OR Pooling╱
          │  │      ╲            ╱
          │  │         │ Y
          │  │         ▼
          │  │  CONVERT L-TH Convolution (Pooling) LAYER    S1403
          │  │  TO Dilated Convolution (Pooling) LAYER
          │  │         │
          │  │         ▼
          │  │  DETERMINE L-TH LAYER DILATE AMOUNT          S1404
          │  │  BASED ON PRODUCT OF STRIDE AMOUNTS
          │  │  FROM 0TH LAYER TO (L-1)TH LAYER OF
          │  │  FCN MODEL AND OUTPUT RESOLUTION
          │  │  OF REGION SEGMENTATION RESULT
          │  │         │
          │  │         ▼
          │  │  DETERMINE L-TH LAYER STRIDE AMOUNT          S1405
          │  │  BASED ON PRODUCT OF STRIDE AMOUNTS
          │  │  FROM 0TH LAYER TO (L-1)TH LAYER OF
          │  │  FCN MODEL AND OUTPUT RESOLUTION
          │  │  OF REGION SEGMENTATION RESULT
          │  │         │
          │  └─────────┤
          │            ▼
          │       INCREMENT L                        S1406
          │            │
          │            ▼
          │  N  ╱ PROCESSING FOR ALL ╲               S1407
          └────╲ LAYERS IS COMPLETED ╱
                ╲                   ╱
                      │ Y
                      ▼
                    END
```

# FIG. 15

Dilated
Convolution
Fy,Fx=3,3
Sy,Sx=1,1
Dy,Dx=1,1

Dilated Pooling
Fy,Fx=2,2
Sy,Sx=1,1
Dy,Dx=1,1

Dilated
Convolution
Fy,Fx=3,3
Sy,Sx=1,1
Dy,Dx=2,2

Dilated Pooling
Fy,Fx=2,2
Sy,Sx=1,1
Dy,Dx=2,2

# FIG. 16

```
        ┌─────────────────────────────────────┐
        │                START                │         S301
        └─────────────────────────────────────┘
                          │
                          ▼
        ┌─────────────────────────────────────┐         S301
        │        ACQUIRE UPPER IMAGE           │
        └─────────────────────────────────────┘
                          │
                          ▼
        ┌─────────────────────────────────────┐         S302
        │      CALCULATE EDGE REGION OF        │
        │   CONTAINER BASED ON UPPER IMAGE     │
        └─────────────────────────────────────┘
                          │
                          ▼
        ┌─────────────────────────────────────┐         S303
        │         DETERMINE TYPE OF            │
        │   CONTAINER BASED ON EDGE REGION     │
        └─────────────────────────────────────┘
                          │
                          ▼
        ┌─────────────────────────────────────┐         S1604
        │  CALCULATE UPPER SURFACE REGION OF   │
        │  COLLECTION TARGET FROM UPPER IMAGE  │
        └─────────────────────────────────────┘
                          │
                          ▼
        ┌─────────────────────────────────────┐         S1605
        │     DETERMINE AMOUNT OF COLLECTION   │
        │          TARGET BASED ON TYPE OF     │
        │  CONTAINER AND UPPER SURFACE REGION  │
        └─────────────────────────────────────┘
                          │
                          ▼
        ┌─────────────────────────────────────┐         S1606
        │     OUTPUT DETERMINATION RESULT      │
        │    OF COLLECTION TARGET AMOUNT       │
        └─────────────────────────────────────┘
                          │
                          ▼
        ┌─────────────────────────────────────┐
        │                 END                 │
        └─────────────────────────────────────┘
```

## FIG. 17

# FIG. 18A

# FIG. 18B

# FIG. 18C

## FIG. 19

| SHORT DIAMETER OF COLLECTION TARGET [mm] | | DISTANCE FROM EDGE OF CONTAINER TO UPPER SURFACE OF COLLECTION TARGET [mm] | | | | | |
|---|---|---|---|---|---|---|---|
| | | 0 | 5 | 10 | ⋯ | 70 | ⋯ |
| TYPE OF CONTAINER | $\alpha$ | 60 | 60 | 60 | | 60 | |
| | $\beta$ | 100 | 100 | 100 | | 50 | |
| | $\gamma$ | 120 | 115 | 105 | | 55 | |
| | $\delta$ | 100 | 100 | 100 | | 100 | |

# FIG. 20

```
          ┌─────────────────────────────────────┐
          │               START                 │
          └─────────────────────────────────────┘
                           │
                           ▼
          ┌─────────────────────────────────────┐  S301
          │        ACQUIRE UPPER IMAGE           │
          └─────────────────────────────────────┘
                           │
                           ▼
          ┌─────────────────────────────────────┐  S302
          │        CALCULATE EDGE REGION OF      │
          │    CONTAINER BASED ON UPPER IMAGE    │
          └─────────────────────────────────────┘
                           │
                           ▼
          ┌─────────────────────────────────────┐  S1604
          │     CALCULATE UPPER SURFACE REGION   │
          │  OF COLLECTION TARGET FROM UPPER IMAGE │
          └─────────────────────────────────────┘
                           │
                           ▼
          ┌─────────────────────────────────────┐  S2005
          │ DETERMINE INCLINATION OF CONTAINER   │
          │   BASED ON EDGE REGION AND UPPER     │
          │            SURFACE REGION            │
          └─────────────────────────────────────┘
                           │
                           ▼
          ┌─────────────────────────────────────┐  S2006
          │      OUTPUT DETERMINATION RESULT     │
          │     OF COLLECTION TARGET AMOUNT      │
          └─────────────────────────────────────┘
                           │
                           ▼
          ┌─────────────────────────────────────┐
          │                END                  │
          └─────────────────────────────────────┘
```

# FIG. 21A

# FIG. 21B

## FIG. 22A

# FIG. 22B

# FIG. 23

IMAGE DATA

103

IMAGE ACQUISITION UNIT — 201

REGION CALCULATION UNIT — 202

LIKELIHOOD ACQUISITION UNIT — 2301

IMAGE SELECTION/DISPLAY UNIT — 2302

TEACHER SIGNAL ACQUISITION UNIT — 2303

LEARNING UNIT — 2304

# FIG. 24

```
                    ┌─────────────────────────────┐
                    │           START             │
                    └─────────────────────────────┘
                                  │
                                  ▼
       ┌─────────────────────────────────────────┐      S2401
   ┌──▶│   SELECT IMAGE BASED ON LIKELIHOOD       │
   │   └─────────────────────────────────────────┘
   │                      │
   │                      ▼
   │   ┌─────────────────────────────────────────┐      S2402
   │   │   ACQUIRE TEACHER SIGNAL BASED           │
   │   │   ON OPERATION OF OPERATOR               │
   │   └─────────────────────────────────────────┘
   │                      │
   │                      ▼
   │            ◇ PREDETERMINED                           S2403
   │  N        ◇  NUMBER OR MORE OF NEW ◇
   └──────────◇  TEACHER IMAGES ARE    ◇
              ◇   COLLECTED            ◇
                        │ Y
                        ▼
       ┌─────────────────────────────────────────┐      S2404
       │   UPDATE COEFFICIENT BY MACHINE          │
       │   LEARNING USING TEACHER IMAGES          │
       └─────────────────────────────────────────┘
                        │
                        ▼
       ┌─────────────────────────────────────────┐      S2405
       │   EVALUATE UPDATED COEFFICIENT           │
       └─────────────────────────────────────────┘
                        │
                        ▼
              ◇ COEFFICIENT IN MEMORY ◇                  S2406
   ┌── N     ◇   IS OVERWRITTEN       ◇
   │          ◇                      ◇
   │                    │ Y
   │                    ▼
   │   ┌─────────────────────────────────────────┐      S2407
   │   │   OVERWRITE COEFFICIENT IN               │
   │   │   MEMORY or STORE COEFFICIENT            │
   │   │   BEFORE AND AFTER UPDATE                │
   │   └─────────────────────────────────────────┘
   │                    │
   │                    ▼
   │   ┌─────────────────────────────────────────┐
   └──▶│            END                           │
       └─────────────────────────────────────────┘
```

# FIG. 25

| | | ASSIGN TEACHER IMAGE | |
| | | COMPLETED | NOT COMPLETED |
|---|---|---|---|
| TYPE OF CONTAINER | CONTAINER α | 14 | 251 |
| | CONTAINER β | 0 | 100 |
| | CONTAINER γ | 30 | 390 |
| | CONTAINER δ | 5 | 200 |
| | UNKNOWN CONTAINER (Φ80,1mm) | 5 | 110 |

*(table title: LIST OF IMAGES)*

# FIG. 26

**DISPLAY TEACHER SIGNAL**

2601

DISPLAY
■ ORIGINAL IMAGE
■ RESULT OF REGION SEGMENTATION
☐ RESULT OF CORRECTION

2602

CORRECTION TOOL
○ BACKGROUND
○ CONTAINER EDGE
○ CONTAINER INNER WALL
○ CONTAINER OUTER WALL
● LIQUID SURFACE

2603

READ  SAVE

2605  2604

# FIG. 27

ASSIGN TEACHER SIGNAL

CONTAINER TYPE: UNKNOWN CONTAINER
INNER DIAMETER: Φ80
THICKNESS: 1mm                    2702

TEACHER SIGNAL:                   2703

2701

READ        SAVE

2605        2604

## INTERNATIONAL SEARCH REPORT

| International application No. |
| --- |
| PCT/JP2020/002860 |

**A. CLASSIFICATION OF SUBJECT MATTER**
Int.Cl. G01N35/10(2006.01)i, G06T7/00(2017.01)i
FI: G01N35/10C, G06T7/00612

According to International Patent Classification (IPC) or to both national classification and IPC

**B. FIELDS SEARCHED**

Minimum documentation searched (classification system followed by classification symbols)
Int.Cl. G01N35/10, G06T7/00

Documentation searched other than minimum documentation to the extent that such documents are included in the fields searched
    Published examined utility model applications of Japan    1922–1996
    Published unexamined utility model applications of Japan    1971–2020
    Registered utility model specifications of Japan    1996–2020
    Published registered utility model applications of Japan    1994–2020

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X<br>Y<br><br>A | JP 2014-500955 A (F. HOFFMANN-LA ROCHE AG)<br>16.01.2014 (2014-01-16), paragraphs [0028]-[0039] | 1-2, 11, 14<br>3-4, 7-10, 12-13<br><br>5-6, 15 |
| Y | JP 2007-298445 A (OLYMPUS CORPORATION) 15.11.2007<br>(2007-11-15), paragraphs [0002]-[0024] | 3 |
| Y | JP 2015-87265 A (HITACHI HIGH-TECHNOLOGIES CORP.)<br>07.05.2015 (2015-05-07), paragraphs [0064]-[0068] | 4 |
| Y | JP 2019-27927 A (HITACHI HIGH-TECHNOLOGIES CORP.)<br>21.02.2019 (2019-02-21), paragraphs [0075],<br>[0148]-[0180] | 7-10 |
| Y | JP 2019-28657 A (PASCO CORPORATION) 21.02.2019<br>(2019-02-21), paragraphs [0008]-[0052] | 9-10 |

☒ Further documents are listed in the continuation of Box C.     ☒ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>  01.04.2020 | Date of mailing of the international search report<br>  14.04.2020 |
| --- | --- |
| Name and mailing address of the ISA/<br>  Japan Patent Office<br>  3-4-3, Kasumigaseki, Chiyoda-ku,<br>  Tokyo 100-8915, Japan | Authorized officer<br><br><br>Telephone No. |

Form PCT/ISA/210 (second sheet) (January 2015)

**INTERNATIONAL SEARCH REPORT**

| International application No. |
| --- |
| PCT/JP2020/002860 |

C (Continuation).    DOCUMENTS CONSIDERED TO BE RELEVANT

| Category* | Citation of document, with indication, where appropriate, of the relevant passages | Relevant to claim No. |
| --- | --- | --- |
| X | JP 2018-146587 A (TSINGHUA UNIVERSITY) 20.09.2018 (2018-09-20), paragraphs [0004]-[0011] | 15 |
| Y | paragraphs [0018]-[0047] | 9-10 |
| Y | JP 2005-304303 A (OLYMPUS CORPORATION) 04.11.2005 (2005-11-04), paragraph [0028] | 12-13 |

Form PCT/ISA/210 (second sheet) (January 2015)

**INTERNATIONAL SEARCH REPORT**

Information on patent family members

International application No.

PCT/JP2020/002860

| | | |
|---|---|---|
| JP 2014-500955 A | 16.01.2014 | US 2013/0315486 A1<br>paragraphs [0057]-[0069]<br>WO 2012/066034 A1<br>EP 2453233 A1<br>CN 103314293 A |
| JP 2007-298445 A | 15.11.2007 | US 2009/0067669 A1<br>paragraphs [0005]-[0038]<br>WO 2007/129639 A1<br>EP 2015035 A1 |
| JP 2015-87265 A | 07.05.2015 | (Family: none) |
| JP 2019-27927 A | 21.02.2019 | WO 2019/026406 A1<br>paragraphs [0076], [0149]-<br>[0181] |
| JP 2019-28657 A | 21.02.2019 | (Family: none) |
| JP 2018-146587 A | 20.09.2018 | US 2018/0260959 A1<br>paragraphs [0005]-[0012],<br>[0024]-[0059]<br>EP 3373246 A1<br>CN 108572183 A |
| JP 2005-304303 A | 04.11.2005 | (Family: none) |

Form PCT/ISA/210 (patent family annex) (January 2015)

**EP 3 955 008 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2004151025 A